# EUROPEAN PATENT APPLICATION

(11) **EP 4 266 393 A1**
(43) Date of publication of application: **25.10.2023**
(21) Application number: 21906713.9
(22) Date of filing: 17.12.2021
(51) Int. Cl.: H01L 51/50, G09F 9/00, G09F 9/30, H01L 27/32, H05B 33/10, H05B 33/26

(54) **ORGANIC ELECTROLUMINESCENT ELEMENT, DISPLAY DEVICE, LIGHTING DEVICE, AND METHOD FOR PRODUCING ORGANIC ELECTROLUMINESCENT ELEMENT**

(30) Priority: 18.12.2020 JP 2020210758; 31.03.2021 JP 2021060032; 20.04.2021 JP 2021071277
(71) Applicant: Nippon Shokubai Co., Ltd., Osaka-shi, Osaka 541-0043 (JP); Nippon Hoso Kyokai, Tokyo 150-8001 (JP)
(72) Inventor: KUWADA, Kenji, Suita-shi, Osaka 564-0034 (JP); MORII, Katsuyuki, Suita-shi, Osaka 564-0034 (JP); HASEGAWA, Munehiro, Suita-shi, Osaka 564-0034 (JP); SASAKI, Tsubasa, Tokyo 157-8510 (JP); FUKAGAWA, Hirohiko, Tokyo 157-8510 (JP); OONO, Taku, Tokyo 157-8510 (JP); SHIMIZU, Takahisa, Tokyo 157-8510 (JP)
(74) Representative: Cabinet Beau de Loménie
(86) International application number: PCT/JP2021/046673
(87) International publication number: WO 2022/131355

(57) **Abstract**

The present invention aims to provide a less expensive flexible organic electroluminescence device having higher characteristics. In particular, the present invention aims to solve at least one of an issue of low-voltage driving and improvement of operational lifetime or an issue of providing an organic electroluminescence device that can exhibit good luminescence and has excellent stability in the atmosphere. The present invention relates to the organic electroluminescence device at least including: a pair of electrodes; and an organic layer between the electrodes, the organic electroluminescence device comprising a metal electron injection layer on the cathode and an organic electron injection layer on the metal electron injection layer, the metal electron injection layer containing a metal species and the organic electron injection layer containing an organic material, being capable of coordination with the metal species.

## Description

### TECHNICAL FIELD

The present invention relates to organic electroluminescence (hereinafter, electroluminescence may be referred to as "EL") devices, displays, lighting systems, and methods for producing an organic electroluminescence device.

### BACKGROUND ART

Organic EL devices are thin, soft, and flexible. Displays including organic EL devices are capable of providing higher brightness, higher definition display than currently dominant liquid crystal displays and plasma displays. Further, displays including organic EL devices have a wider viewing angle than liquid crystal displays. Thus, displays including organic EL devices are expected to be widely used, for example, as displays of TVs and mobiles. In addition, organic EL devices are also expected to be used in lighting systems.

An organic EL device is a stack of a cathode, an emitting layer, and an anode. In the organic electroluminescence device that excites a light-emitting organic compound with energy generated upon recombination of holes injected from the anode with electrons injected from the cathode to achieve emission, smooth hole injection from the anode and smooth electron injection from the cathode are important. For more smooth hole injection and more smooth electron injection, various materials for the hole injection layer and the electron injection layer have been studied. Recently, a conventional organic electroluminescence device using polyethylenimine or a compound modified with polyethylenimine has been reported as a material that can provide an electron injection layer by coating (Non-Patent Literatures 1 to 3).

Non-Patent Literatures 4 to 6 have reported that phenanthroline derivatives, which contain nitrogen atoms, have a high coordination ability with metal ions and thus facilitate electron emission from metal atoms, and greatly lower electron injection barriers at the interface between an electrode and an organic layer. They have also reported that use of a phenanthroline derivative between the electrode and the organic layer improves the electron injection ability. Also, Patent Literatures 1 to 3 disclose organic EL devices including an electron injection layer formed from a compound having a structure in which a nitrogen-containing amino group is attached to a group derived from an electron transport compound directly or via another group or an organic material having an acid dissociation constant pKa of 1 or greater.

An organic electroluminescence device in which the layers between the cathode and the anode are all formed from organic compounds is prone to degradation due to oxygen and water in principle. Such a device has to be strictly sealed to prevent entry of oxygen and water. This complicates the production of organic electroluminescence devices and makes the production of flexible devices difficult.

In response to this issue, a hybrid organic-inorganic light-emitting diode device (HOILED device) has been proposed (Patent Literature 4) which has an inverted structure in which one or some of the layers between the cathode and the anode are an inorganic oxide layer(s) and in which the cathode is on the substrate. In the device, use of a hole transport layer and an electron transport layer each made of an inorganic oxide enables use of a conductive oxide electrode such as FTO or ITO as a cathode and use of gold as an anode. This means that any electrode can be used for driving the device. As a result, use of metal having a low work function, such as an alkali metal or an alkali metal compound, is no longer required and luminescence can be achieved without strict sealing, greatly contributing to production of flexible devices. This HOILED device has been expected to develop to be an inverted organic EL device.

### CITATION LIST

### -Patent Literature

Patent Literature 1: JP 2017-157743 A
Patent Literature 2: JP 2019-176093 A
Patent Literature 3: JP 2019-179863 A
Patent Literature 4: JP 2009-70954 A

### -Non-Patent Literature

Non-Patent Literature 1: Tao Xiong and three others, "Applied Physics Letters", Vol. 93, 2008, pp. 123310-1
Non-Patent Literature 2: Yinhua Zhou and twenty-one others, "Science", Vol. 336, 2012, p. 327
Non-Patent Literature 3: Jianshan Chen and six others, "Journal of Materials Chemistry", 2012, Vol. 22, p. 5164
Non-Patent Literature 4: Hiroyuki Yoshida, "JOURNAL OF PHYSICAL CHEMISTRY" (J. Phys. Chem. C20151194324459-24464), Vol. 119, 2015, p. 24459
Non-Patent Literature 5: Zhengyang Bin and seven others, "Nature Communications", Vol. 10, 2019, p. 866
Non-Patent Literature 6: Hirohiko Fukagawa and eight others, "Nature Communications", Vol. 11, 2020, p. 1
[Non-Patent Literature 7: Herbert B Michaelson, "J. Appl. Phys", Vol. 48, 1977, p. 4729

### SUMMARY OF INVENTION

### -Technical Problem

The present invention has been made in view of such a current state of the art and aims to provide a less expensive flexible organic electroluminescence device having higher characteristics. In particular, the present invention can solve at least one of an issue of low-voltage driving and improvement of operational lifetime or an issue of providing an organic electroluminescence device that can exhibit good luminescence and has excellent stability in the atmosphere.

### -Solution to Problem

The present invention has been made by studies with a focus on using an organic material having a coordinating site such as an organic material capable of multidentate coordination having a heterocyclic ring containing an electron-donating nitrogen atom as a material for the electron injection layer of an organic electroluminescence device, and on a metal species to which the organic material is to be coordinated. Furthermore, in consideration of stability in the atmosphere, a metal with a high work function is used as the metal element of the metal species or a small amount of material not having a high work function is allowed to work, thereby materializing a flexible device. As a result of these studies, it is found that providing a metal layer on the cathode layer for the coordination reaction can produce an organic electroluminescence device having a longer lifetime, compared to a conventional technique using an oxide. The mechanism is thought to be that coordination bonds are denser in the metal layer than in the oxide layer, and thereby the injection barrier can be significantly and uniformly reduced and oxygen and water entering from the outside can be reduced.

In other words, the cathode in the organic electroluminescence device of the present invention may be made of any material, specifically, any material having necessary functions (e.g., a transparent material having stability in the atmosphere). Specifically, a thin metal atom layer (metal electron injection layer) capable of undergoing coordinating reaction is formed on the cathode for electron injection, and an organic layer (organic electron injection layer) made of an organic substance that serves as the corresponding ligand is formed directly thereon. The metal electron injection layer in this embodiment is limited only by the coordination ability, not by the work function. Thus, a metal species that is stable in the atmosphere can be selected. Considering the degree of freedom in selecting the cathode material, it is possible to produce an organic electroluminescence device in which the cathode and the electron injection layer, which are most susceptible to deterioration by water and oxygen, are stable in the atmosphere.

Furthermore, the present inventors have made various studies on materials for improving the electron injection ability of devices in order to develop organic electroluminescence devices capable of exhibiting good luminescence. Then, the present inventors have found that an organic electroluminescence device that can exhibit good luminescence and have excellent stability in the atmospheric can be obtained in the following way: as the material of the electron injection layer, an organic material having a coordinating site such as an organic material capable of multidentate coordination having a heterocyclic ring containing an electron-donating nitrogen atom is used; and an organic electron injection layer containing the organic material is formed adjacent to a cathode having a predetermined film thickness containing magnesium to which the organic material is to be coordinated and at least one of silver or aluminum.

The present invention has been completed based on the above findings, and the following describes the summary thereof.
[1] An organic electroluminescence device at least including:
   a pair of electrodes; and
   an organic layer between the electrodes,
   the organic electroluminescence device including
   a metal electron injection layer on the cathode and an organic electron injection layer on the metal electron injection layer,
   the metal electron injection layer containing a metal species and the organic electron injection layer containing an organic material, the metal species and the organic material being capable of coordination.
[2] The organic electroluminescence device according to [1],
   wherein the metal species, or when the metal species includes multiple metal species, all the metal species in the metal electron injection layer has a work function of 4.0 eV or higher.
[3] The organic electroluminescence device according to [1] or [2],
   wherein the metal electron injection layer contains at least one selected from the group consisting of zinc, copper, and aluminum.
[4] The organic electroluminescence device according to any one of [1] to [3],
   wherein the metal electron injection layer has a thickness of 5 nm or less.
[5] The organic electroluminescence device according to any one of [1] to [4],
   wherein the organic material in the organic electron injection layer is a compound having a nitrogen-containing substituent.
[6] The organic electroluminescence device according to any one of [1] to [5],
   wherein the organic material in the organic electron injection layer is a compound having a fused ring structure containing nitrogen-containing heterocyclic ring.
[7] The organic electroluminescence device according to any one of [1] to [6],
   wherein the organic material in the organic electron injection layer is a compound having a structure represented by the following formula (1): 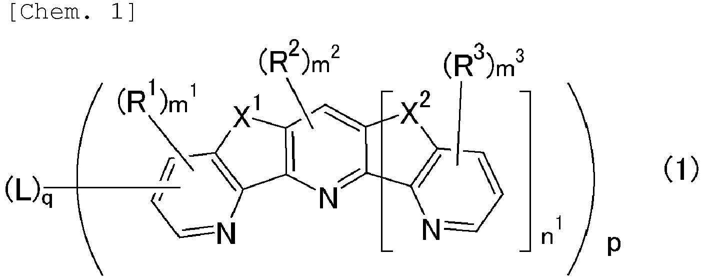 wherein X¹ and X² are the same as or different from each other and are each a nitrogen atom optionally having a substituent, an oxygen atom optionally having a substituent, a sulfur atom optionally having a substituent, or a divalent linking group optionally having a substituent; L is a direct bond or a linking group having a valence of p; n¹ is the number of 0 or 1; p is the number of 1 to 4; q is the number of 0 or 1, with q being 0 when p is 1; R¹ to R³ are the same as or different from each other and are each a monovalent substituent; m¹ to m³ are the same as or different from each other and are each the number of 0 to 3; R¹ to R³ are each optionally combined with X¹ or X² to form a ring structure; and when R¹ includes multiple R¹'s, R¹'s are optionally combined to each other to form a ring structure, with the same applying to R² and R³.
[8] The organic electroluminescence device according to any one of [1] to [6],
   wherein the organic material in the organic electron injection layer is a compound having a structure, as a skeletal structure, represented by any of the following formulas (5-66) to (5-68): 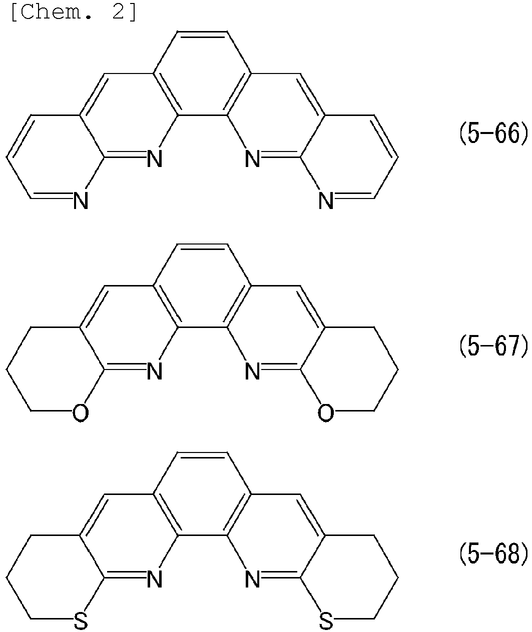
[9] The organic electroluminescence device according to any one of [1] to [6],
   wherein the organic material in the organic electron injection layer is a hexahydropyrimidopyrimidine compound having a structure represented by the following formula (6) : 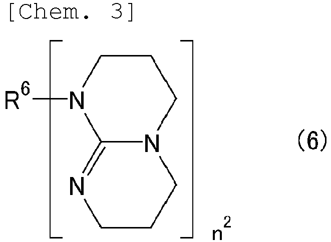 wherein R⁶ is an aromatic hydrocarbon group optionally having a substituent, an aromatic heterocyclic group optionally having a substituent, an arylalkylene group optionally having a substituent, a divalent to tetravalent acyclic or cyclic hydrocarbon group optionally having a substituent, a group of a combination of two or more of these groups, or a group of a combination of a nitrogen atom and one or more of these groups; and n² is an integer of 1 to 4.
[10] The organic electroluminescence device according to any one of [1] to [9],
   wherein the cathode has a work function of 4.0 eV or higher.
[11] The organic electroluminescence device according to any one of [1] to [10],
   wherein the cathode is on a substrate.
[12] The organic electroluminescence device according to any one of [1] to [3] and [5] to [11],
   wherein the metal electron injection layer is obtained by mixing with a cathode constituent material.
[13] An organic electroluminescence device at least including:
   a pair of electrodes; and
   an organic layer between the electrodes,
   the cathode being a layer having an average thickness of 40 nm or less and containing magnesium and at least one of silver or aluminum, with a percentage by volume of magnesium being 2% or higher and 30% or lower,
   the organic electroluminescence device comprising
   an organic electron injection layer being adjacent to the cathode and containing an organic material capable of coordinating to a metal atom.
[14] The organic electroluminescence device according to [13],
   wherein the organic material in the organic electron injection layer is a compound having a nitrogen-containing substituent.
[15] The organic electroluminescence device according to [13] or [14],
   wherein the organic material in the organic electron injection layer is a compound having a fused ring structure containing nitrogen-containing heterocyclic ring.
[16] The organic electroluminescence device according to any one of [13] to [15],
   wherein the organic material in the organic electron injection layer is a compound having a structure represented by the following formula (1): 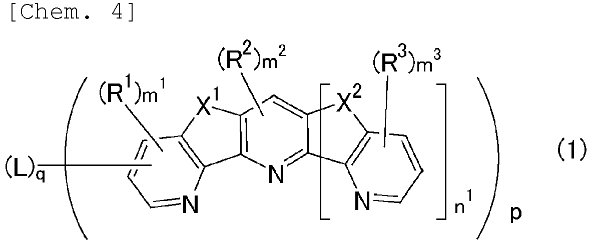 wherein X¹ and X² are the same as or different from each other and are each a nitrogen atom optionally having a substituent, an oxygen atom optionally having a substituent, a sulfur atom optionally having a substituent, or a divalent linking group optionally having a substituent; L is a direct bond or a linking group having a valence of p; n¹ is the number of 0 or 1; p is the number of 1 to 4; q is the number of 0 or 1, with q being 0 when p is 1; R¹ to R³ are the same as or different from each other and are each a monovalent substituent; m¹ to m³ are the same as or different from each other and are each the number of 0 to 3; R¹ to R³ are each optionally combined with X¹ or X² to form a ring structure; and when R¹ includes multiple R¹'s, R¹'s are optionally combined to each other to form a ring structure, with the same applying to R² and R³.
[17] The organic electroluminescence device according to any one of [13] to [15],
   wherein the organic material in the organic electron injection layer is a compound having a structure, as a skeletal structure, represented by any of the following formulas (5-66) to (5-68): 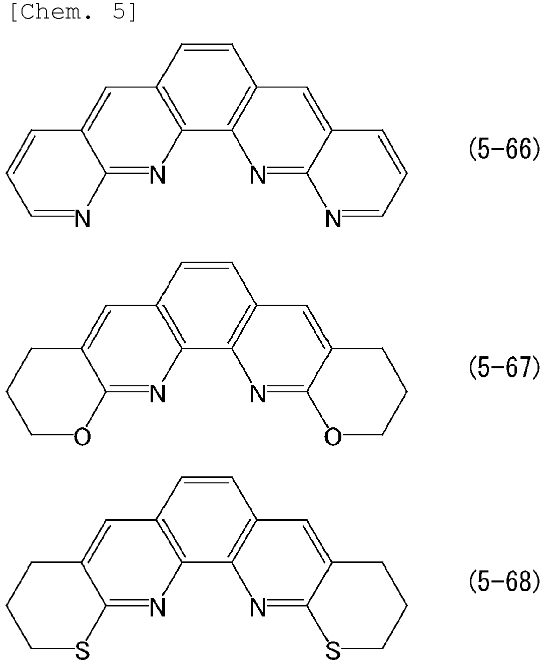
[18] The organic electroluminescence device according to any one of [13] to [15],
   wherein the organic material in the organic electron injection layer is a hexahydropyrimidopyrimidine compound having a structure represented by the following formula (6) : 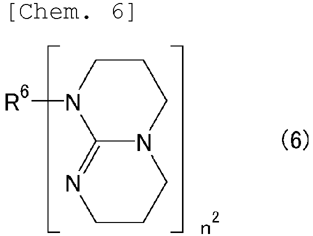 wherein R⁶ is an aromatic hydrocarbon group optionally having a substituent, an aromatic heterocyclic group optionally having a substituent, an arylalkylene group optionally having a substituent, a divalent to tetravalent acyclic or cyclic hydrocarbon group optionally having a substituent, a group of a combination of two or more of these groups, or a group of a combination of a nitrogen atom and one or more of these groups; and n² is an integer of 1 to 4.
[19] The organic electroluminescence device according to any one of [13] to [18],
   wherein the cathode is on a substrate.
[20] A display including the organic electroluminescence device according to any one of [1] to [19] .
[21] A lighting system including the organic electroluminescence device according to any one of [1] to [19] .
[22] A method for producing an organic electroluminescence device including at least a pair of electrodes and an organic layer between the electrodes, the method including:
   forming a metal electron injection layer adjacent to the cathode and containing a metal species; and
   forming on the metal electron injection layer an organic electron injection layer containing an organic material capable of coordinating to the metal species in the metal electron injection layer.
[23] A method for producing an organic electroluminescence device including at least a pair of electrodes and an organic layer between the electrodes, the method including:
   forming a cathode having an average thickness of 40 nm or less and containing magnesium and at least one of silver or aluminum, a percentage by volume of magnesium being 2% or higher and 30% or lower; and
   forming on the cathode an organic electron injection layer containing an organic material capable of coordinating to a metal atom in the cathode.

### -Advantageous Effects of Invention

The organic electroluminescence device of the present invention satisfies at least one of a feature of low-voltage driving and excellent operational lifetime or a feature of good luminescence and excellent stability in the atmosphere, and thus can be used as a flexible organic electroluminescence device for a display, a lighting system, and a light source.

### BRIEF DESCRIPTION OF DRAWINGS

FIG. 1 is a schematic view of a stacked structure of an organic electroluminescence device of the present invention.
FIG. 2 is a schematic view of a stacked structure of an organic electroluminescence device produced in Example 1.
FIG. 3 is a schematic view of a stacked structure of an organic electroluminescence device produced in Example 5.
FIG. 4 is a schematic view of a stacked structure of an organic electroluminescence device produced in Example 9.
FIG. 5 is a schematic view of a stacked structure of an organic electroluminescence device produced in Example 11.
FIG. 6 is a schematic view of a stacked structure of an organic electroluminescence device produced in Example 12.
FIG. 7 is a schematic view of an exemplary stacked structure of the organic electroluminescence device of the present invention.
FIG. 8 is a schematic view of an exemplary stacked structure of the organic electroluminescence device of the present invention.
FIG. 9 is a schematic view of an exemplary stacked structure of the organic electroluminescence device of the present invention.
FIG. 10 is a schematic view of a stacked structure of an organic electroluminescence device produced in Example 13.
FIG. 11 is a schematic view of a stacked structure of an organic electroluminescence device produced in Comparative Example 8.
FIG. 12 shows the voltage-luminance characteristics of the organic electroluminescence device produced in Example 1.
FIG. 13 shows the lifetime characteristics of the organic electroluminescence device produced in Example 1.
FIG. 14 shows the voltage-luminance characteristics of an organic electroluminescence device produced in Example 2.
FIG. 15 shows the lifetime characteristics of the organic electroluminescence device produced in Example 2.
FIG. 16 shows the voltage-luminance characteristics of an organic electroluminescence device produced in Example 3.
FIG. 17 shows the lifetime characteristics of the organic electroluminescence device produced in Example 3.
FIG. 18 shows the voltage-luminance characteristics of an organic electroluminescence device produced in Example 4.
FIG. 19 shows the lifetime characteristics of the organic electroluminescence device produced in Example 4.
FIG. 20 shows the voltage-luminance characteristics of an organic electroluminescence device produced in Comparative Example 1.
FIG. 21 shows the lifetime characteristics of the organic electroluminescence device produced in Comparative Example 1.
FIG. 22 shows the voltage-luminance characteristics of organic electroluminescence devices produced in Examples 5 and 6 and Comparative Example 2.
FIG. 23 shows the lifetime characteristics of the organic electroluminescence devices produced in Examples 5 and 6 and Comparative Example 2.
FIG. 24 shows the voltage-luminance characteristics of organic electroluminescence devices produced in Examples 7 and 8 and Comparative Example 3.
FIG. 25 shows the voltage-luminance characteristics of the organic electroluminescence device produced in Example 9.
FIG. 26 shows the current density-efficiency characteristics of the organic electroluminescence device produced in Example 9.
FIG. 27 shows the lifetime characteristics of the organic electroluminescence device produced in Example 9.
FIG. 28 shows the results obtained as follows: the organic electroluminescence device produced in Example 9 is allowed to stand in the atmosphere without sealing, and the states of luminescence at the initial stage, after 22 hours, and after 90 hours are observed.
FIG. 29 shows the voltage-luminance characteristics of an organic electroluminescence device produced in Example 10.
FIG. 30 shows the current density-efficiency characteristics of the organic electroluminescence device produced in Example 10.
FIG. 31 shows the lifetime characteristics of the organic electroluminescence device produced in Example 10.
FIG. 32 shows the results obtained as follows: the organic electroluminescence device produced in Example 10 is allowed to stand in the atmosphere without sealing, and the states of luminescence at the initial stage, after 22 hours, and after 90 hours are observed.
FIG. 33 shows the voltage-luminance characteristics of an organic electroluminescence device produced in Comparative Example 4.
FIG. 34 shows the current density-efficiency characteristics of the organic electroluminescence device produced in Comparative Example 4.
FIG. 35 shows the lifetime characteristics of the organic electroluminescence device produced in Comparative Example 4.
FIG. 36 shows the results obtained as follows: the organic electroluminescence device produced in Comparative Example 4 is allowed to stand in the atmosphere without sealing, and the states of luminescence at the initial stage, after 22 hours, and after 90 hours are observed.
FIG. 37 shows the voltage-luminance characteristics of the organic electroluminescence device produced in Example 11.
FIG. 38 shows the current density-efficiency characteristics of the organic electroluminescence device produced in Example 11.
FIG. 39 shows the lifetime characteristics of the organic electroluminescence device produced in Example 11.
FIG. 40 shows the results obtained as follows: the organic electroluminescence device produced in Example 11 is allowed to stand in the atmosphere without sealing, and the states of luminescence at the initial stage, after 22 hours, and after 90 hours are observed.
FIG. 41 shows the evaluation results of the stability in the atmosphere of the organic electroluminescence device produced in Example 12 stored in an environment with a temperature of 85°C and a humidity of 85%.
FIG. 42 shows the evaluation results of the stability in the atmosphere of an organic electroluminescence device produced in Example 12 stored in an environment with a temperature of 85°C and a humidity of 85%.
FIG. 43 shows the observation results of the state of luminescence of the organic electroluminescence device produced in Example 13 that has been stored in an environment with a temperature of 85°C and a humidity of 85% for 65 hours without sealing.
FIG. 44 shows the observation results of the state of luminescence of an organic electroluminescence device produced in Example 14 that has been stored in an environment with a temperature of 85°C and a humidity of 85% for 65 hours without sealing.
FIG. 45 shows the observation results of the state of luminescence of an organic electroluminescence device produced in Example 15 that has been stored in an environment with a temperature of 85°C and a humidity of 85% for 65 hours without sealing.
FIG. 46 shows the observation results of the state of luminescence of an organic electroluminescence device produced in Example 16 that has been stored in an environment with a temperature of 85°C and a humidity of 85% for 65 hours without sealing.
FIG. 47 shows the observation results of the state of luminescence of an organic electroluminescence device produced in Comparative Example 6 that has been stored in an environment with a temperature of 85°C and a humidity of 85% for 65 hours without sealing.
FIG. 48 shows the observation results of the state of luminescence of an organic electroluminescence device produced in Comparative Example 8 that has been stored in an environment with a temperature of 85°C and a humidity of 85% for 65 hours without sealing.
FIG. 49 shows the voltage-luminance characteristics of an organic electroluminescence device produced in Example 18.
FIG. 50 shows the observation results of the state of luminescence of the organic electroluminescence device produced in Example 18 that has been stored in an environment with a temperature of 85°C and a humidity of 85% for 65 hours without sealing.

### DESCRIPTION OF EMBODIMENTS

The organic electroluminescence device of the present invention will be specifically described layer by layer below.

### "Organic EL element"

The present invention includes (1) an organic electroluminescence device (organic EL device) including: an organic layer including an emitting layer between a cathode and an anode; a layer including a metal electron injection layer, which is a metal thin film, adjacent to the cathode and an organic electron injection layer containing an organic material which coordinates to a metal element in the metal layer, and (2) an organic electroluminescence device (organic EL device) including: an organic layer including an emitting layer between a cathode and an anode, the cathode being a layer having an average thickness of 40 nm or less and containing magnesium and at least one of silver or aluminum, with the percentage by volume of magnesium being 2% or higher and 30% or lower; and an organic electron injection layer being adjacent to the cathode and containing an organic material capable of coordinating to a metal atom.

Hereinafter, the organic EL device (1) is referred to as an organic EL device according to a first aspect of the present invention, and the organic EL device (2) is referred to as an organic EL device according to a second aspect of the present invention. Both are collectively referred to as an organic EL device of the present invention.

The structure of the organic EL device according to the second aspect of the present invention can provide excellent electron injection properties owing to the coordination bonds between magnesium and the organic material capable of coordinating to the metal atom. Further, use of a layer having an average thickness of 40 nm or less and containing at least one of silver or aluminum and a small proportion of magnesium as the cathode can provide a device having excellent stability in the atmosphere while magnesium, whose work function is small, is used. Furthermore, the device having such a structure has a longer lifetime than the previously described device including a metal oxide layer on the cathode. The mechanism is thought to be that coordination bonds are denser in magnesium than in the oxide layer, and thereby the injection barrier can be significantly and uniformly reduced and oxygen and water entering from the outside can be reduced.

The organic EL device of the present invention preferably has an inverted structure in which the cathode is on the substrate, but may have a conventional structure in which the anode is on the substrate. The organic EL device of the present invention may be a bottom emission device in which light is extracted from the substrate or may be a top emission device in which light is extracted from the side away from the substrate.

The following specifically describes the organic EL device of the present invention with examples.

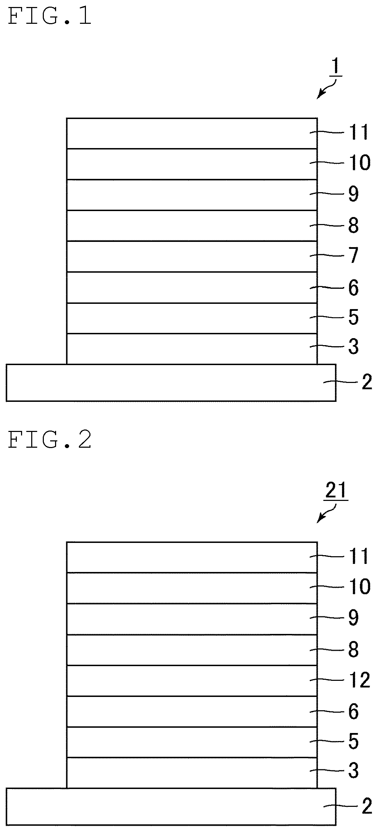

FIG. 1 is a schematic cross-sectional view of an exemplary stacked structure of the organic electroluminescence device according to the first aspect of the present invention. Here, the structure of an inverted organic EL device is described. An organic EL device 1 of the present embodiment shown in FIG. 1 includes an emitting layer 8 between a cathode 3 and an anode 11. In the organic EL device 1 shown in FIG. 1, a metal electron injection layer 5 and an organic electron injection layer 6, which are features of the present invention, are adjacent to each other and are disposed between the cathode 3 and the emitting layer 8. The organic EL device 1 optionally includes an electron transport layer 7 and a hole blocking layer 12. A hole transport layer 9 and a hole injection layer 10 are disposed between the emitting layer 8 and the anode 11.

Other examples of the structure of the organic EL device according to the first aspect of the present invention include the structures shown in FIGS. 2 to 6.

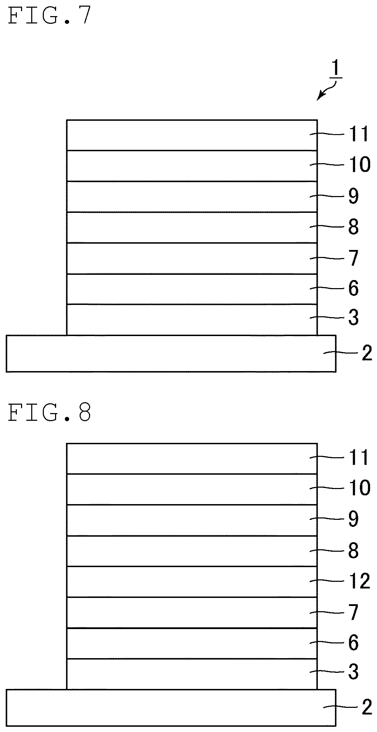

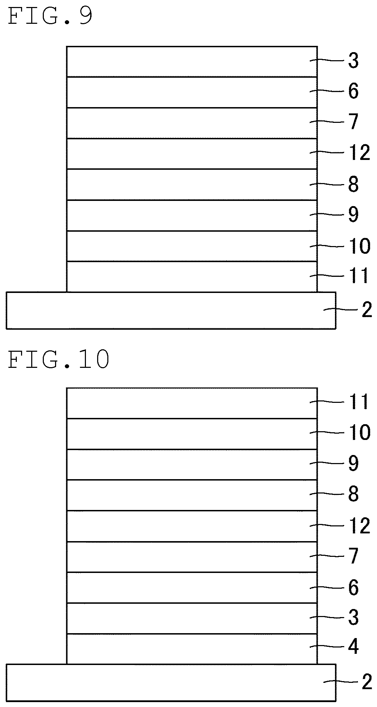

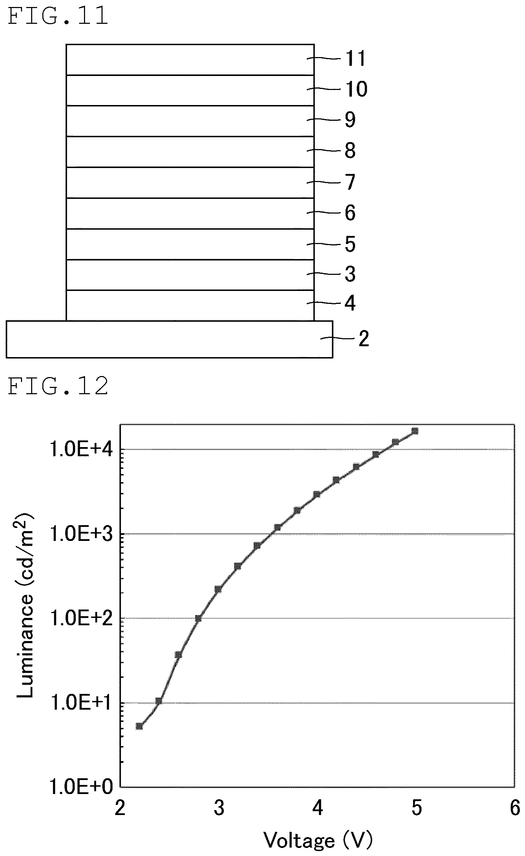

FIGS. 7 and 8 are each a schematic cross-sectional view of an exemplary stacked structure of the organic EL device according to the second aspect of the present invention. Here, the structure of an inverted organic EL device is described. Organic EL devices 1 of the present embodiment shown in FIGS. 7 and 8 each include an emitting layer 8 between a cathode 3 and an anode 11. In the organic EL devices 1 shown in FIGS. 7 and 8, an organic electron injection layer 6 is disposed between the cathode 3 and the emitting layer 8, with the organic electron injection layer 6 being adjacent to the cathode 3. The organic EL elements 1 each optionally include an electron transport layer 7 and a hole blocking layer 12. A hole transport layer 9 and a hole injection layer 10 are disposed between the emitting layer 8 and the anode 11. The organic EL devices 1 each optionally include a metal oxide layer 4 as a base for the cathode 3 in order to increase the stability of the cathode (FIGS. 10 and 11). Another example of the structure of the organic EL device according to the second aspect of the present invention is the structure shown in FIG. 9.

In the present embodiment, an example of an inverted organic EL device 1 will be described, but the organic EL device of the present invention may have a conventional structure in which the anode is disposed between the substrate and the emitting layer.

When the organic EL device according to the first aspect of the present invention has a conventional structure, it also has a stacked structure including the organic electron injection layer and the metal electron injection layer adjacent to each other between the cathode and the emitting layer like an inverted organic EL device.

When the organic EL device according to the second aspect of the present invention has a conventional structure, it also includes the organic electron injection layer between the cathode and the emitting layer, with the organic electron injection layer being adjacent to the cathode, like an inverted organic EL device.

The following describes the materials and thicknesses of the layers defining the organic EL device 1 and a sealing method of the organic EL device 1, which can also be applied to a conventional organic EL device, except for the materials of the cathode and the anode described below, as for the organic EL device according to the first aspect of the present invention. Only in the top emission inverted organic EL device, the thickness of the metal electron injection layer described later is not limited.

As for the organic EL device according to the second aspect of the present invention, the materials and thicknesses of the layers defining the organic EL device 1 and the sealing method of the organic EL device 1 can also be applied to a conventional organic EL device, except for the material of the anode described below.

The layers defining the organic EL device of the present invention are described below.

### "Substrate"

The substrate 2 is made of, for example, a resin material or a glass material.

Examples of the resin material of the substrate 2 include polyethylene terephthalate, polyethylene naphthalate, polypropylene, cycloolefin polymers, polyamides, polyethersulfone, polymethylmethacrylate, polycarbonate, and polyarylate. A resin material is preferred for the substrate 2 because it makes the organic EL device 1 to be highly flexible.

Examples of the glass material of the substrate 2 include silica glass and soda glass.

When the organic EL device 1 is a bottom emission device, a transparent substrate may be used as the material of the substrate 2.

When the organic EL device 1 is a top emission device, the substrate 2 may be a transparent substrate or an opaque substrate. Examples of the opaque substrate include a substrate made of a ceramic material such as alumina, a substrate in which an oxide film (insulating film) is formed on the surface of a metal plate such as a stainless steel plate, and a substrate made of a resin material.

The average thickness of the substrate 2 may be determined according to, for example, the material of the substrate 2, and is preferably 0.1 to 30 mm, more preferably 0.1 to 10 mm.

The average thickness of the substrate 2 can be measured with a digital multimeter or a caliper.

### "Cathode"

The cathode 3 is in direct contact with the substrate 2.

In the organic EL device according to the first aspect of the present invention, the material of the cathode 3 is preferably a material having a work function of 4.0 eV or higher. Examples thereof include conductive oxide materials such as indium tin oxide (ITO), indium zinc oxide (IZO), fluorine tin oxide (FTO), In₃O₃, SnO₂, Sb-containing SnO₂, and Al-containing ZnO and silver and silver alloys. Preferred among these materials of the cathode 3 are ITO, IZO, FTO, and silver.

In the organic EL device according to the first aspect of the present invention, the average thickness of the cathode 3 is not limited, and is preferably 10 to 500 nm, more preferably 100 to 200 nm. When the cathode 3 is made of silver, the average thickness of the cathode 3 is preferably 5 to 40 nm, more preferably 10 to 20 nm.

In the organic EL device according to the second aspect of the present invention, the cathode 3 is a layer containing magnesium and at least one of silver or aluminum, with each of which being an elemental metal. The cathode 3 may contain either one or both of silver and aluminum. In the cathode 3, the percentage by volume of magnesium in the layer containing magnesium and at least one of silver or aluminum is 30% or lower. With such a percentage, the device has excellent electron injection properties and excellent stability in the atmosphere. The percentage by volume of magnesium is preferably 20% or lower, more preferably 10% or lower, even more preferably 5% or lower. The percentage by volume of magnesium is 2% or higher from the viewpoint of achieving better electron injection properties, preferably 3% or higher, more preferably 4% or higher.

In the organic EL device according to the second aspect of the present invention, the cathode 3 has an average thickness of 40 nm or less. With such a thickness, the device has excellent stability in the atmosphere. The average thickness of the cathode 3 is preferably 35 nm or less, more preferably 25 nm or less. From the viewpoint of the conductivity of electrodes and variation in production of devices, the average thickness of the cathode 3 is preferably 3 nm or more, more preferably 5 nm or more, even more preferably 10 nm or more.

The average thickness of the cathode 3 can be measured with a stylus profiler or a spectroscopic ellipsometer.

When the organic EL device of the present invention has a conventional structure in which the anode is disposed between the substrate and the emitting layer, these materials may be used as a material for the anode. In this case, the average thickness of the anode is preferably the same as that of the cathode 3.

The work function is referred to the values described in Non-Patent Literature 7.

### "Metal electron injection layer"

In the organic EL device according to the first aspect of the present invention, the metal electron injection layer 5 is formed from a metal species that serves as the central metal of a metal complex formed with the material of the organic electron injection layer as a ligand. Thus, the metal electron injection layer 5 may be a thin film in terms of functions. When the organic EL device is a bottom emission device, which requires transparency, the thickness of the metal electron injection layer 5 is preferably 0.1 to 5 nm, more preferably 0.5 to 2 nm. When the organic EL device is a top emission device, which requires no transparency, the thickness of the metal electron injection layer 5 is not limited.

The average thickness of the metal electron injection layer 5 can be measured with a stylus profiler or a spectroscopic ellipsometer.

The material in the metal electron injection layer 5 is a simple substance of a metal species having a coordination ability. From the viewpoint of stability in the atmosphere, the material preferably has a work function of 4.0 eV or higher.

The metal electron injection layer 5 may be a metal layer consisting of a single metal element; a stack of layers each consisting of a combination of two or more different metal elements, a stack of layers each consisting of a single metal element, or a stack of layers each consisting of a combination of two or more different metal elements and a layer(s) consisting of a single metal element; or a layer consisting of a combination of two or more different metal elements. The metal electron injection layer may also serve as a cathode.

Examples of the metal element in the metal electron injection layer 5 include copper, nickel, palladium, platinum, gold, cobalt, zinc, aluminum, chromium, manganese, iron, tin, indium, titanium, zirconium, vanadium, niobium, tantalum, molybdenum, tungsten, indium, gallium, and cadmium.

The work function is referred to the values described in Non-Patent Literature 7.

When the metal electron injection layer 5 includes a layer consisting of a combination of two or more different metal elements, at least one of the metal elements is preferably aluminum, zinc, or copper.

When the metal electron injection layer 5 is a layer consisting of a single metal element, the layer is preferably made of a metal selected from the group consisting of aluminum, zinc, and copper.

### "Organic electron injection layer"

In the organic EL device according to the first aspect of the present invention, the organic electron injection layer 6 forms a complex with the metal electron injection layer 5 to improve electron injection.

In the organic EL device according to the second aspect of the present invention, the organic electron injection layer 5 forms a complex with magnesium in the cathode 3 to improve electron injection.

The material in the organic electron injection layer 6 may be any organic material having a coordination ability. In particular, the organic material in the organic electron injection layer 6 is preferably a compound having a nitrogen-containing substituent, more preferably a compound having a fused ring structure of nitrogen-containing heterocyclic rings. An example of the organic material in the organic electron injection layer in the present invention is a compound having a structure represented by the following formula (1): 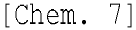 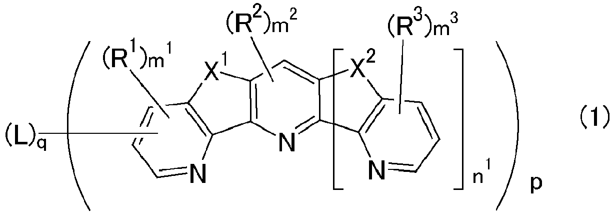 wherein X¹ and X² are the same as or different from each other and are each a nitrogen atom optionally having a substituent, an oxygen atom optionally having a substituent, a sulfur atom optionally having a substituent, or a divalent linking group optionally having a substituent; L is a direct bond or a linking group having a valence of p; n¹ is the number of 0 or 1; p is the number of 1 to 4; q is the number of 0 or 1, with q being 0 when p is 1; R¹ to R³ are the same as or different from each other and are each a monovalent substituent; m¹ to m³ are the same as or different from each other and are each the number of 0 to 3; R¹ to R³ are each optionally combined with X¹ or X² to form a ring structure; and when R¹ includes multiple R¹'s, R¹'s are optionally combined to each other to form a ring structure, with the same applying to R² and R³.

x¹ and X² in the formula (1) are the same as or different from each other and are each a nitrogen atom optionally having a substituent, an oxygen atom optionally having a substituent, a sulfur atom optionally having a substituent, or a divalent linking group optionally having a substituent.

Examples of the divalent linking group include a divalent hydrocarbon group and a group in which one or some of the carbon atoms of a hydrocarbon group are replaced with a heteroatom selected from a nitrogen atom, an oxygen atom, and a sulfur atom.

The hydrocarbon group preferably has a carbon number of 1 to 6, more preferably has a carbon number of 1, 2, or 6.

The hydrocarbon group may be linear, branched, or cyclic, or may include a combination of two or more of these structures.

The divalent hydrocarbon group may be a saturated hydrocarbon group such as an alkylene group or may be an unsaturated hydrocarbon group such as an alkenylene group or an alkynylene group.

Specifically, the divalent hydrocarbon group is preferably any of compounds represented by the formulas (2-1) to (2-4) below. R in the formulas (2-1) to (2-4) is a substituent. Specific examples of R in the formulas (2-1) to (2-4) and a substituent in X¹ and X² include monovalent substituents for R¹ to R³ described later.

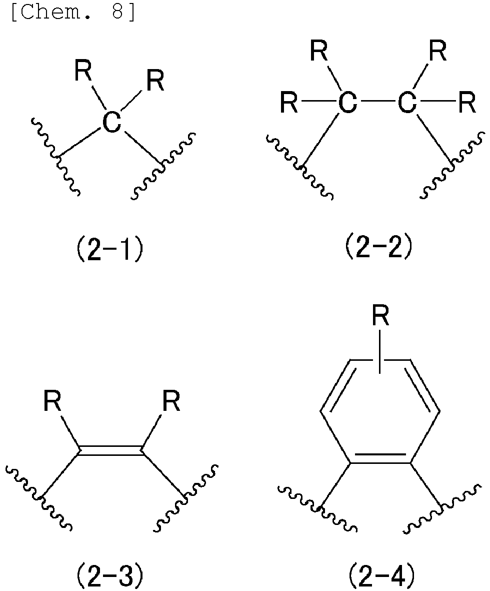

L in the formula (1) is a direct bond or a linking group having a valence of p. L is a direct bond only when p is 2.

Examples of the linking group having a valence of p include nitrogen, oxygen, sulfur, and carbon atoms, and a group prepared by removing p number of hydrogen atoms from a hydrocarbon group or a group in which one or some of the carbon atoms of a hydrocarbon group are replaced with a heteroatom selected from a nitrogen atom, an oxygen atom, and a sulfur atom as.

When the linking group having a valence of p contains a carbon atom, it preferably has a carbon number of 1 to 30. More preferably, it has a carbon number of 1 to 20.

The hydrocarbon group may be linear, branched, or cyclic, or may include a combination of two or more of these structures.

The hydrocarbon group may be any of a saturated hydrocarbon group, an unsaturated hydrocarbon group, and an aromatic hydrocarbon group.

Examples of the aromatic hydrocarbon group include groups prepared by removing a hydrogen atom from an aromatic compound such as a benzene ring, a naphthalene ring, an anthracene ring, a tetracene ring, a pentacene ring, a triphenylene ring, a pyrene ring, a fluorene ring, or an indene ring.

R¹ to R³ in the formula (1) are the same as or different from each other, and are each a monovalent substituent.

Examples of the monovalent substituent include a fluorine atom; haloalkyl groups such as fluoromethyl, difluoromethyl, and trifluoromethyl groups; C1-C20 linear or branched acyclic alkyl groups such as methyl, ethyl, propyl, isopropyl, butyl, isobutyl, and tert-butyl groups; C5-C7 cyclic alkyl groups such as cyclopentyl, cyclohexyl, and cycloheptyl groups; C1-C20 linear or branched acyclic alkoxy groups such as methoxy, ethoxy, propoxy, isopropoxy, butoxy, isobutoxy, tert-butoxy, pentyloxy, hexyloxy, heptyloxy, and octyloxy groups; a nitro group; a cyano group; C1-C10 alkyl group-containing alkylamino groups such as methylamino, ethylamino, dimethylamino, and diethylamino groups; cyclic amino groups such as pyrrolidino, piperidino, and morpholino group; diarylamino groups such as diphenylamino and carbazolyl groups; acyl groups such as acetyl, propionyl, and butyryl groups; C2-C30 alkenyl groups such as a styryl group; a C5-C20 aryl group optionally substituted with a halogen atom such as a fluorine atom, a C1-C20 alkyl group, a C1-C20 alkoxy group, a C1-C20 amino group, or the like (specific examples of the aryl group include the above-described examples of the aromatic hydrocarbon group); a C4-C40 heterocyclic group containing at least one of a nitrogen atom, a sulfur atom, or an oxygen atom, optionally substituted with a halogen atom such as a fluorine atom, a C1-C20 alkyl group, a C1-C20 alkoxy group, a C1-C20 amino group, or the like (the heterocyclic group may consist of one ring or may be a compound in which multiple compounds each consisting of one aromatic heterocyclic ring are directly bound to each other via a carbon-carbon bond, or a fused heterocyclic group. Specific examples of the heterocyclic group include aromatic heterocyclic groups such as a thiophene ring, a furan ring, a pyrrole ring, a benzothiophene ring, a benzofuran ring, an indole ring, a dibenzothiophene ring, a dibenzofuran ring, a carbazole ring, a thiazole ring, a benzothiazole ring, an oxazole ring, a benzoxazole ring, an imidazole ring, a benzimidazole ring, a pyridine ring, a pyrimidine ring, a pyrazine ring, a pyridazine ring, a triazine ring, a quinoline ring, an isoquinoline ring, a quinoxaline ring, a benzothiadiazole ring, and a phenanthridine ring); ester groups and thioether groups; and combinations of these. These groups may be substituted with a halogen atom, a heteroatom, an alkyl group, or an aromatic ring, for example.

The compound represented by the formula (1) may be a compound having a structure represented by the formula (3) below containing one phenanthroline. This compound is also preferred as a material of the organic electron injection layer in the present invention.

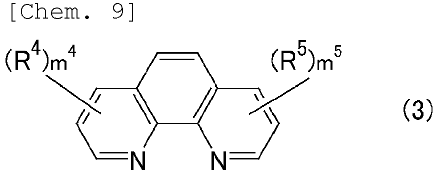

In the formula (3), R⁴ and R⁵ are the same as or different from each other and are each a dialkylamino group or an alkoxy group; and m⁴ and m⁵ are the same as or different from each other and are each the number of 1 or 2. When R⁴ includes multiple R⁴'s, R⁴' s may be combined to each other to form a ring structure. The same shall apply to R⁵.

R⁴ and R⁵ in the formula (3) are the same as or different from each other and are each a dialkylamino group or an alkoxy group.

Preferably, the dialkylamino group has a C1-C20 alkyl group such as a methyl group or an ethyl group. More preferably, the dialkylamino group has a C1-C10 alkyl group. The carbon numbers of the two alkyl groups in the dialkylamino group may be the same as or different from each other. An amino group in which two alkyl groups link to each other such as a cyclic amino group (e.g., a piperidino group, a pyrrolidino group, or a morpholino group) is also preferred.

Examples of the alkoxy group include the alkoxy groups for R¹ to R³ in the formula (1).

In addition to the compounds having one phenanthroline, compounds represented by the following formulas (4-1) to (4-4) each having multiple phenanthrolines are considered to be effective for generating a negative charge.

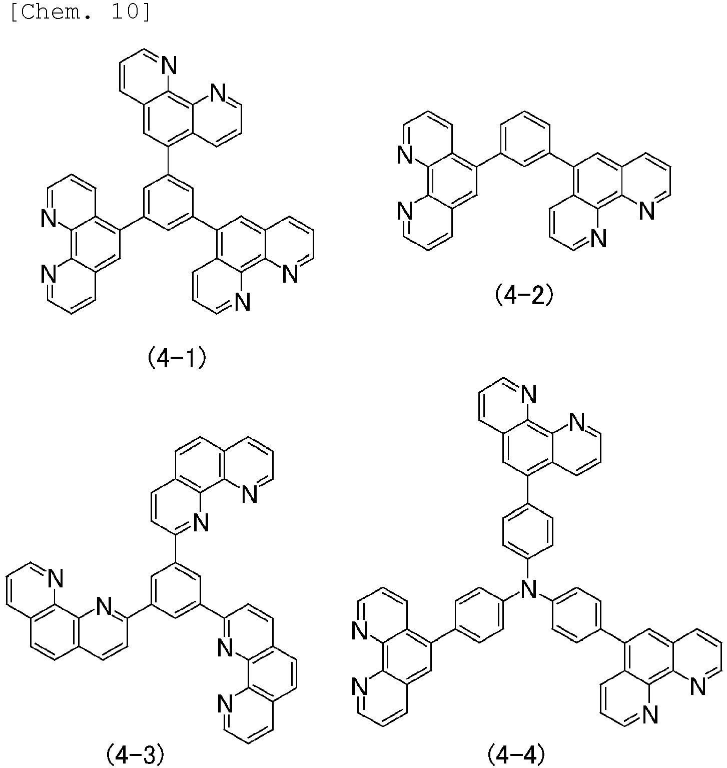

The letter p in the formula (1) is the number of 1 to 4, preferably the number of 1 to 3. Specific examples of the compound represented by the formula (1) in which p is 1 include compounds represented by the following formulas (5-1) to (5-9):

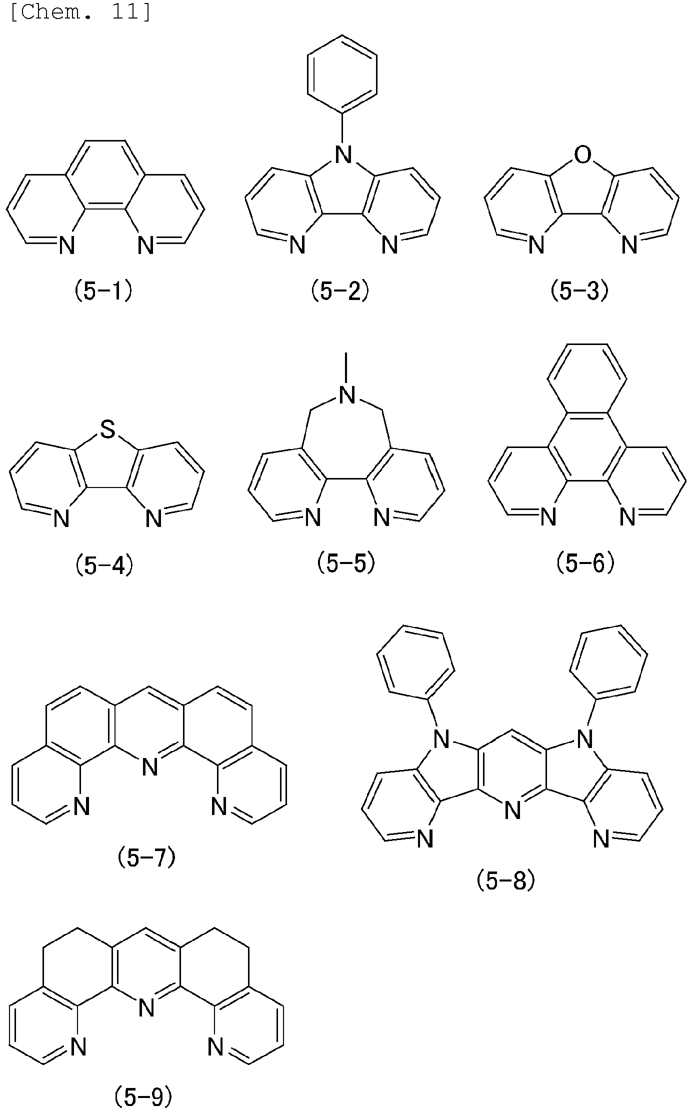

The letter n¹ in the formula (1) is the number of 0 or 1. In a preferred embodiment of the present invention, the compound represented by the formula (1) is a compound represented by the formula (1) in which n¹ is 0. Specific examples of the compound represented by the formula (1) in which n¹ is 0 include compounds represented by the formulas (5-1) to (5-6).

The compounds represented by the formulas (5-10) to (5-65) below, which are included in compounds represented by the formula (1) and have structures similar to the above compounds, are also preferred.

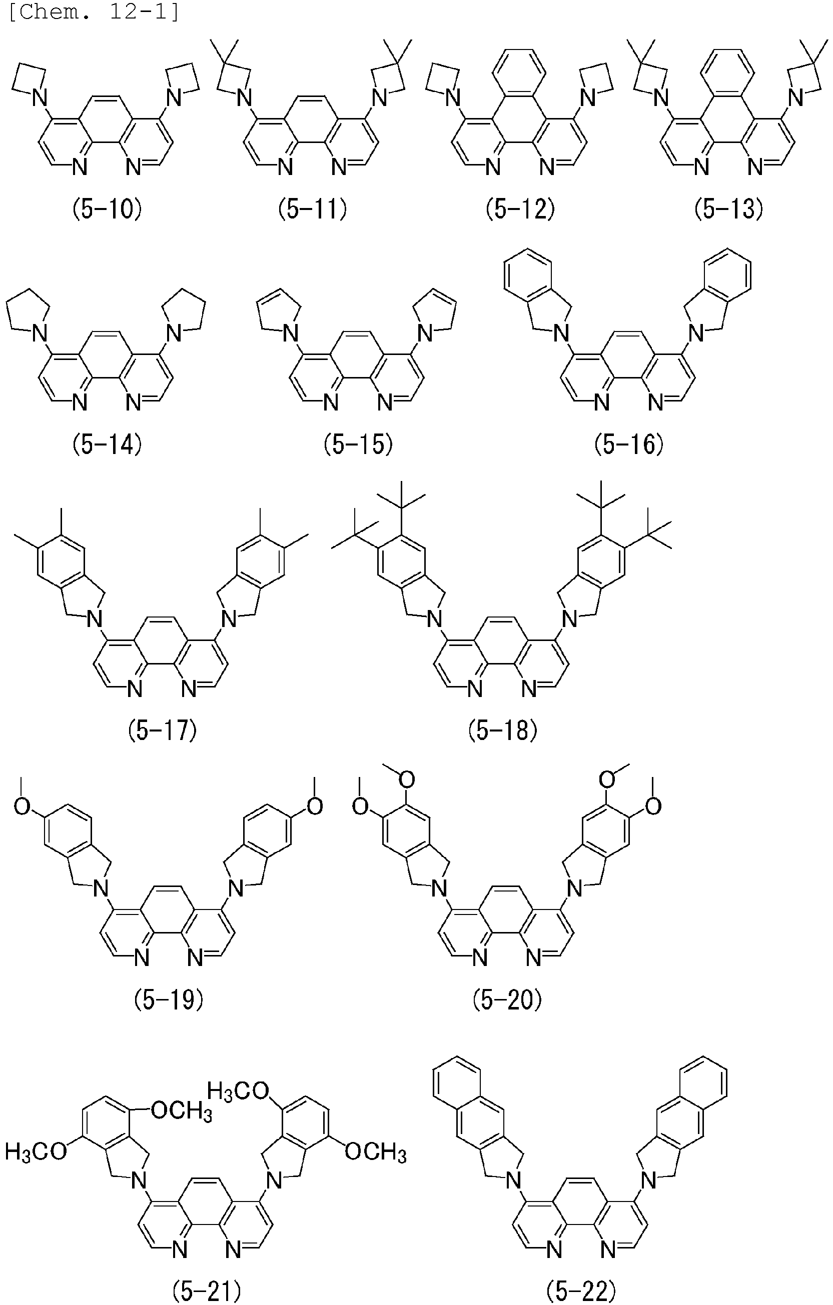

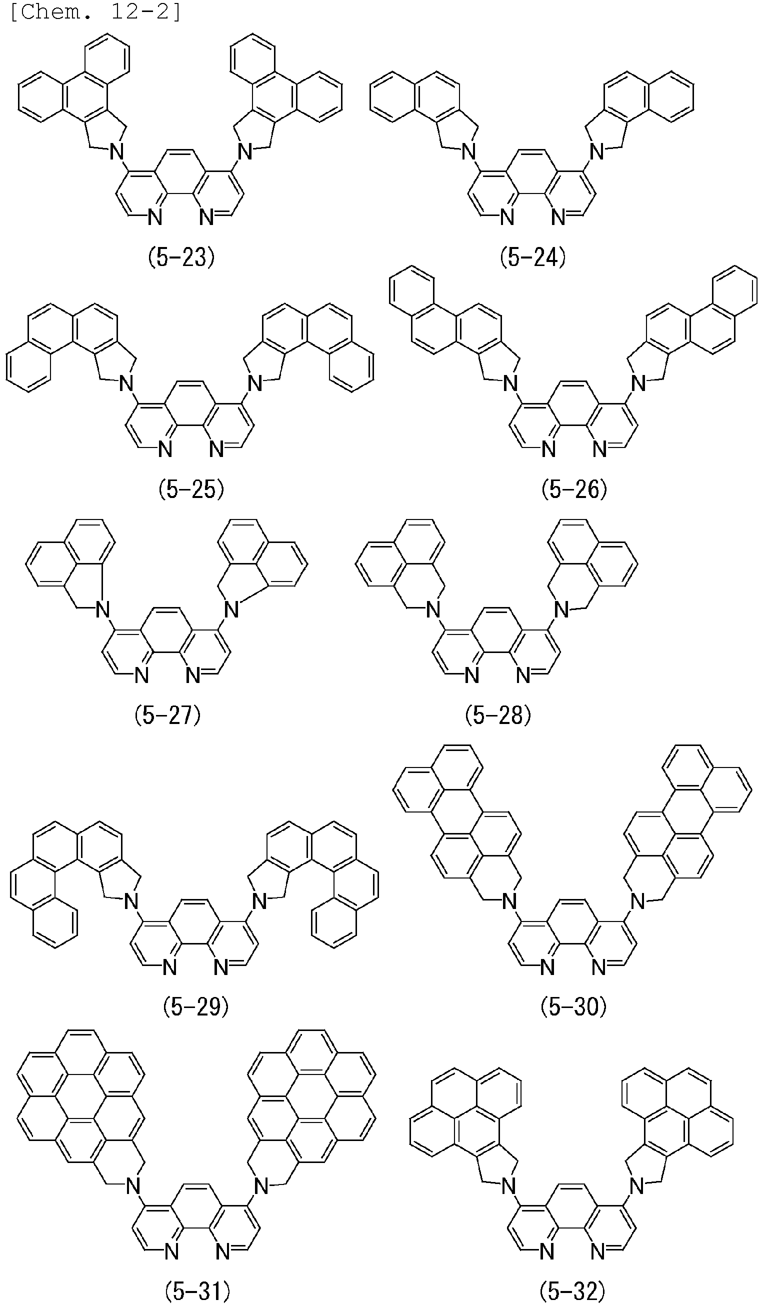

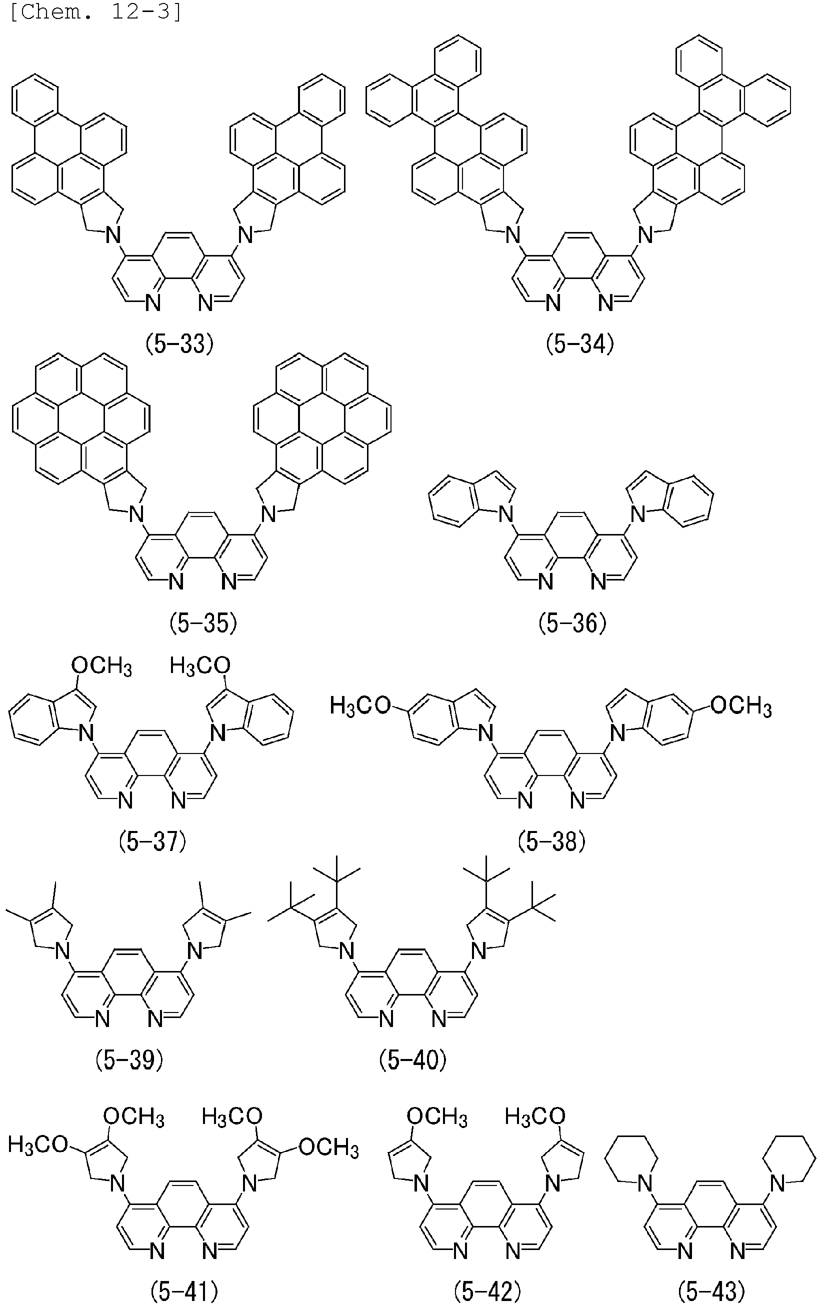

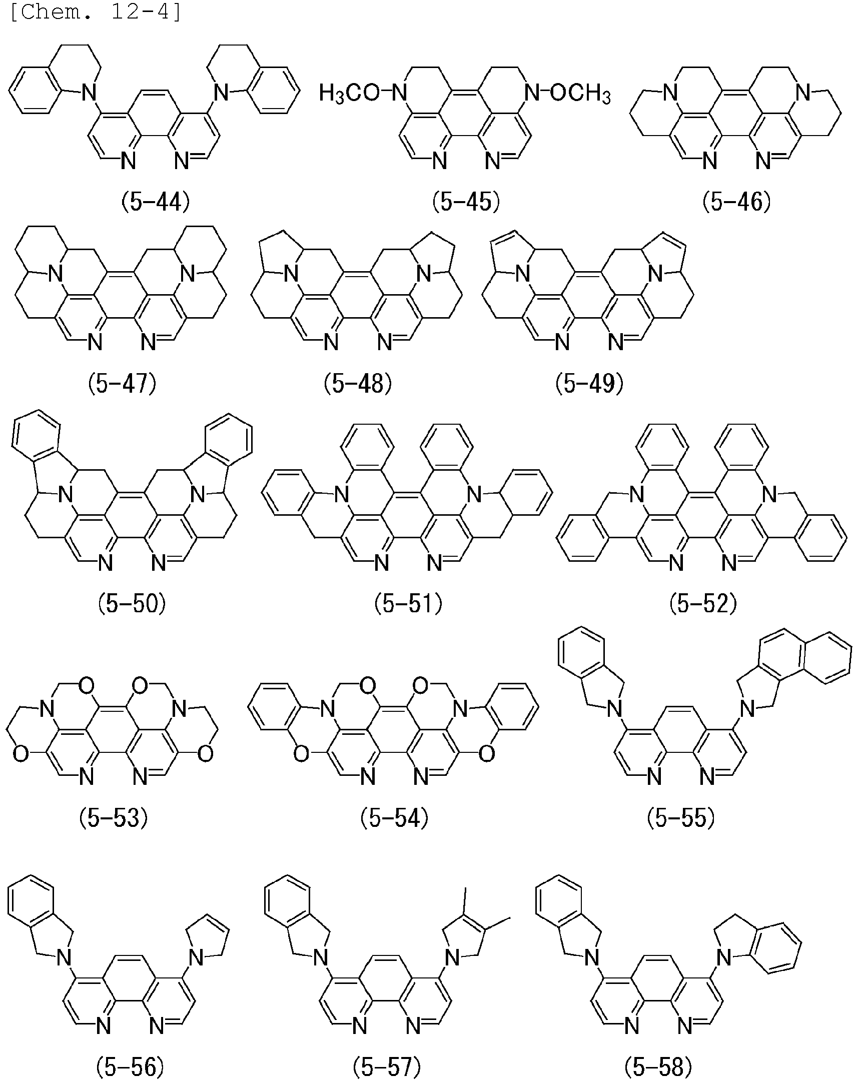

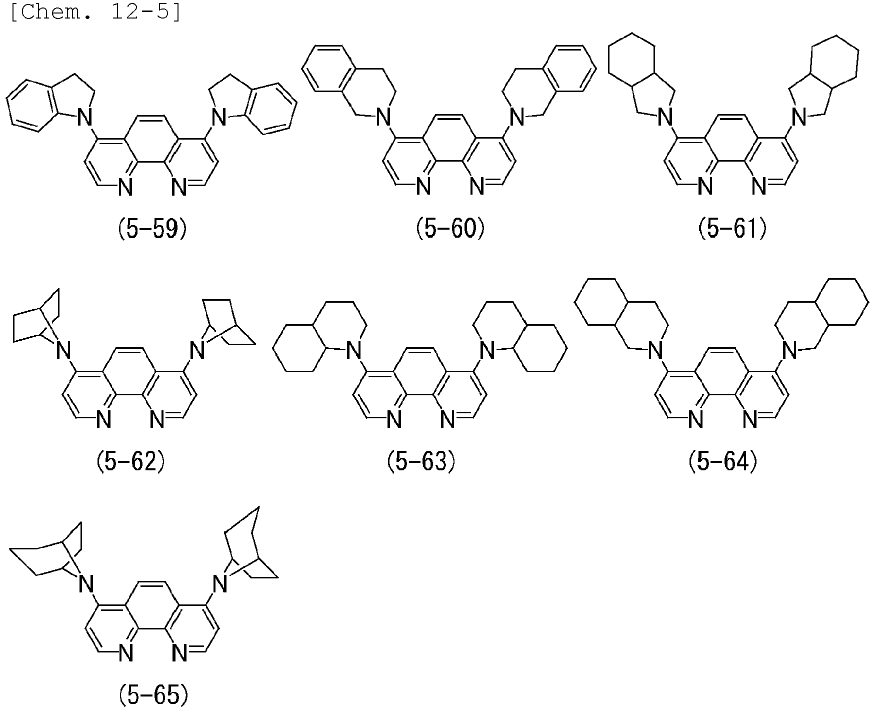

Furthermore, the material of the organic electron injection layer 6 may be any compound containing coordinatable nitrogen atoms. The material may be any of various compounds having any of the structures represented by the formulas (5-66) to (5-68) below as a skeletal structure. In addition to the compounds having any of the structures represented by the formulas (5-66) to (5-68) below, the material may be any of compounds having any of the structures represented by the formulas (5-66) to (5-68) each having a substituent. Examples of the substituent include those mentioned for R¹ to R³ in the formula (1). These structures each may have one substituent or two or more substituents. When two or more substituents are contained, these substituents may be bound to each other to form a ring structure.

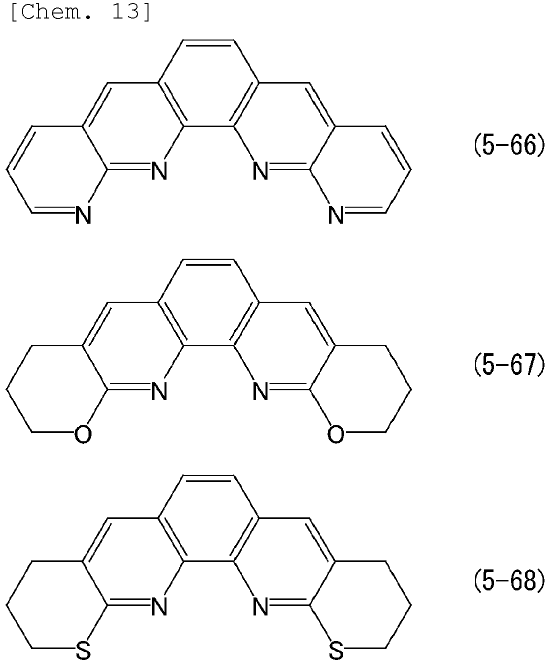

Furthermore, the material of the organic electron injection layer 6 may also be a hexahydropyrimidopyrimidine compound having a structure represented by the following formula (6): 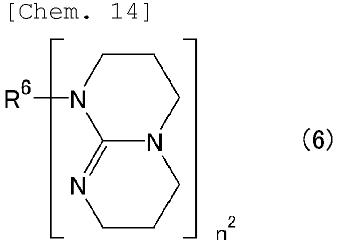 wherein R⁶ is an aromatic hydrocarbon group optionally having a substituent, an aromatic heterocyclic group optionally having a substituent, an arylalkylene group optionally having a substituent, a divalent to tetravalent acyclic or cyclic hydrocarbon group optionally having a substituent, a group of a combination of two or more of these groups, or a group of a combination of a nitrogen atom and one or more of these groups; and n² is an integer of 1 to 4.

The hexahydropyrimidopyrimidine compound having the structure represented by the formula (6) can coordinate to the metal species in the metal electron injection layer 5 in the organic EL device according to the first aspect of the present invention and can coordinate to magnesium in the cathode 3 in the organic EL device according to the second aspect of the present invention. Thereby, the reaction at the interface with oxygen or water that enters from the outside is prevented, and the stability in the atmosphere of the devices is increased. Furthermore, the hexahydropyrimidopyrimidine compound interacts with the metal species in the metal electron injection layer 5 in the organic EL device according to the first aspect of the present invention to generate dipoles, improving the electron injection properties of the metal electron injection layer 5. Also, the hexahydropyrimidopyrimidine compound interacts with magnesium in the cathode 3 in the organic EL device according to the second aspect of the present invention to generate dipoles, improving the electron injection properties of the cathode 3.

In the formula (6), R⁶ is an aromatic hydrocarbon group optionally having a substituent, an aromatic heterocyclic group optionally having a substituent, an arylalkylene group optionally having a substituent, a divalent to tetravalent acyclic or cyclic hydrocarbon group optionally having a substituent, a group of a combination of two or more of these groups, or a group of a combination of a nitrogen atom and one or more of these groups.

The aromatic hydrocarbon group and the aromatic heterocyclic group each preferably have a carbon number of 3 to 30, more preferably 4 to 24, even more preferably 5 to 20.

Examples of the aromatic hydrocarbon group include a compound consisting of one aromatic ring (e.g., benzene); a compound in which multiple aromatic rings are directly bound to each other via a carbon-carbon bond (e.g., biphenyl or diphenylbenzene); and a group prepared by removing 1 to 4 hydrogen atoms from any of aromatic rings of a fused cyclic aromatic hydrocarbon compound such as naphthalene, anthracene, phenanthrene, or pyrene.

Examples of the aromatic heterocyclic group include a compound consisting of one aromatic heterocyclic ring (e.g., thiophene, furan, pyrrole, oxazole, oxadiazole, thiazole, thiadiazole, imidazole, pyridine, pyrimidine, pyrazine, or triazine); a compound in which any two or more of the compounds each consisting of one aromatic heterocyclic ring are directly bound to each other via a carbon-carbon bond (e.g., bipyridine); and a group prepared by removing 1 to 4 hydrogen atoms from any of the aromatic heterocyclic rings of a fused cyclic heteroaromatic hydrocarbon compound such as quinoline, quinoxaline, benzothiophene, benzothiazole, benzimidazole, benzoxazole, indole, carbazole, dibenzofuran, dibenzothiophene, acridine, or phenanthroline.

The arylalkylene group may be a group of a combination of any of the aromatic hydrocarbon groups and a C1-C3 alkylene group.

The divalent to tetravalent acyclic or cyclic hydrocarbon group preferably has a carbon number of 1 to 12, more preferably 1 to 6, even more preferably 1 to 4. The acyclic hydrocarbon group may be linear or branched.

R⁶ may be a group of a combination of two or more groups selected from the aromatic hydrocarbon groups, the aromatic heterocyclic groups, the arylalkylene groups, and the divalent to tetravalent acyclic hydrocarbon groups.

R⁶ may also be a group of a combination of a nitrogen atom and one or more groups selected from the aromatic hydrocarbon groups, the aromatic heterocyclic groups, the arylalkylene groups, and the divalent to tetravalent acyclic hydrocarbon groups. Examples of these groups include groups prepared by removing 1 to 4 hydrogen atoms from triphenylamine or a trialkylamine such as trimethylamine.

The aromatic hydrocarbon group, the aromatic heterocyclic group, and the arylalkylene group may each contain one or more monovalent substituents.

Examples of the monovalent substituent include those mentioned as the specific examples of the monovalent substituent for R¹ to R³ in the formula (1).

The letter n² in the formula (6) is an integer of 1 to 4, preferably 2 or 3.

Specific examples of the hexahydropyrimidopyrimidine compound having a structure represented the formula (6) include compounds represented by the following formulas (7-1) to (7-34).

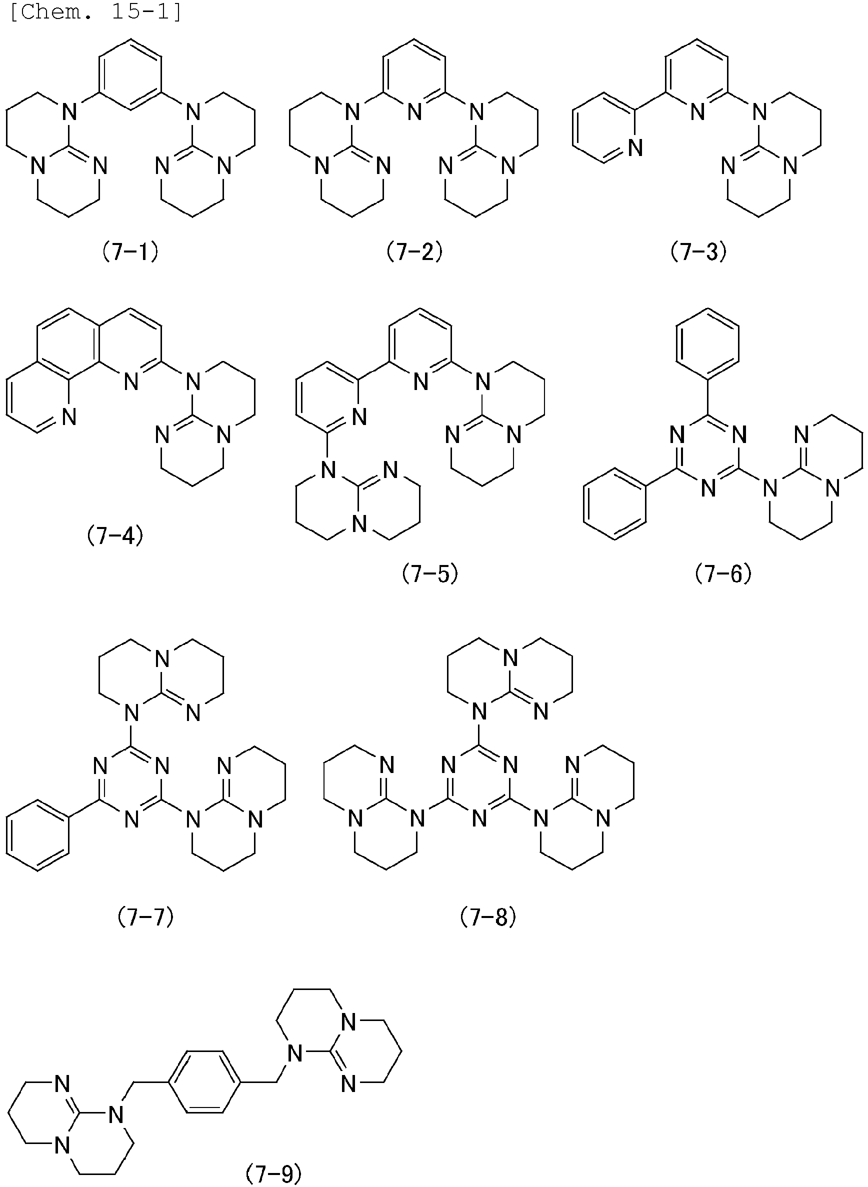

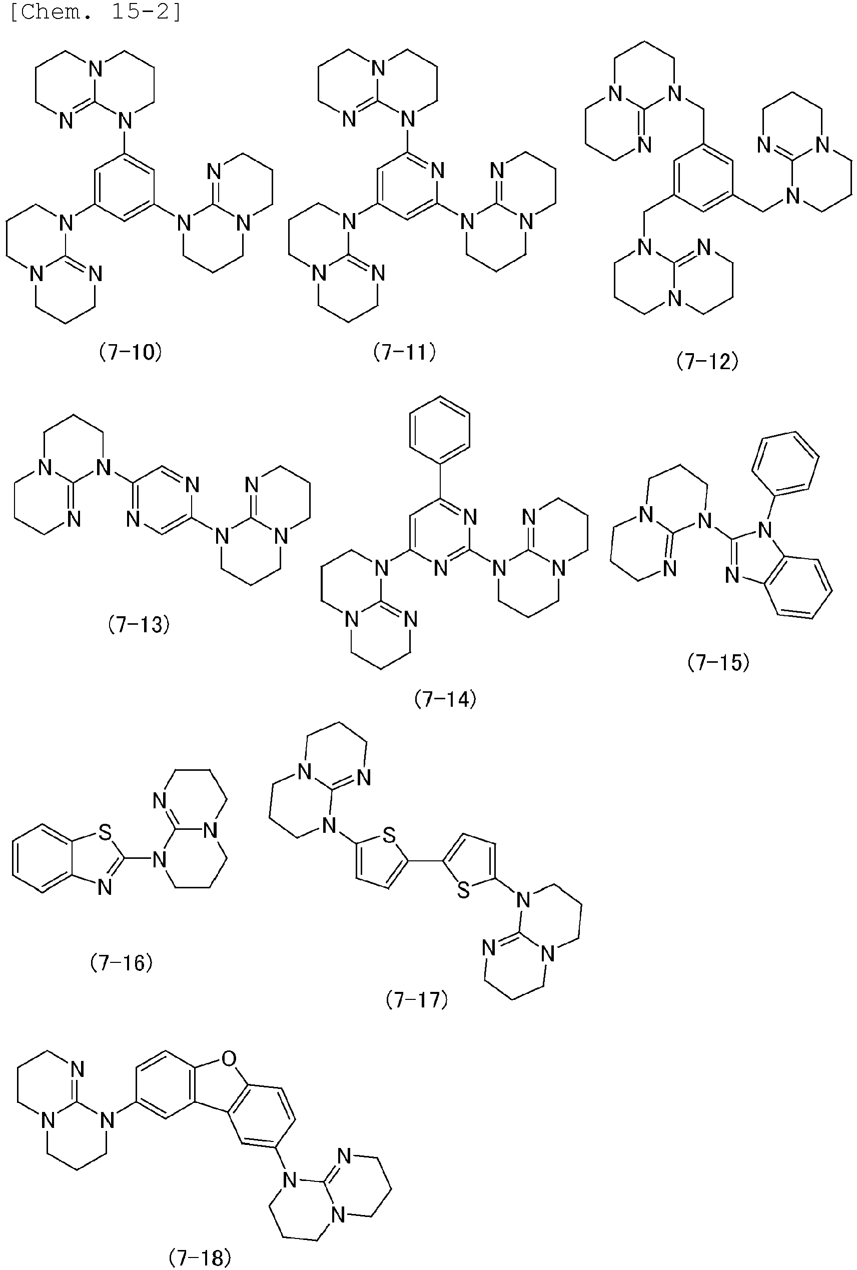

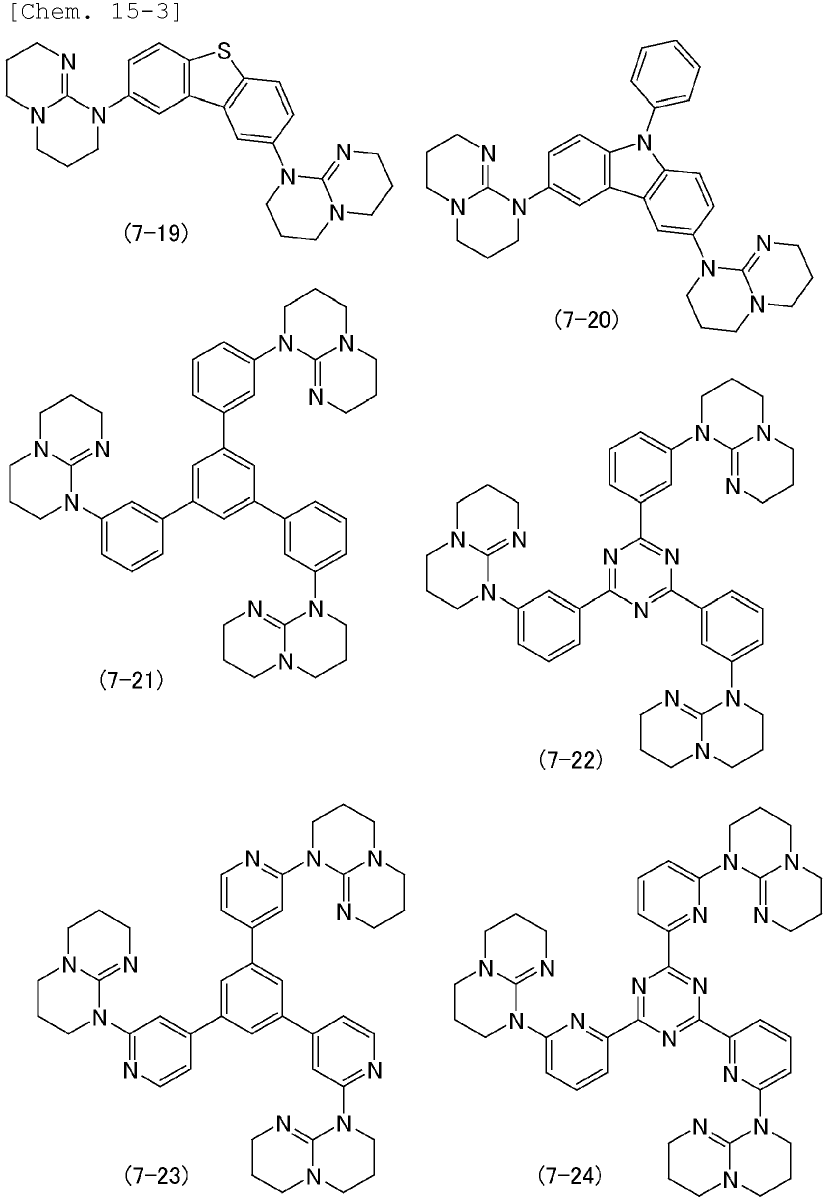

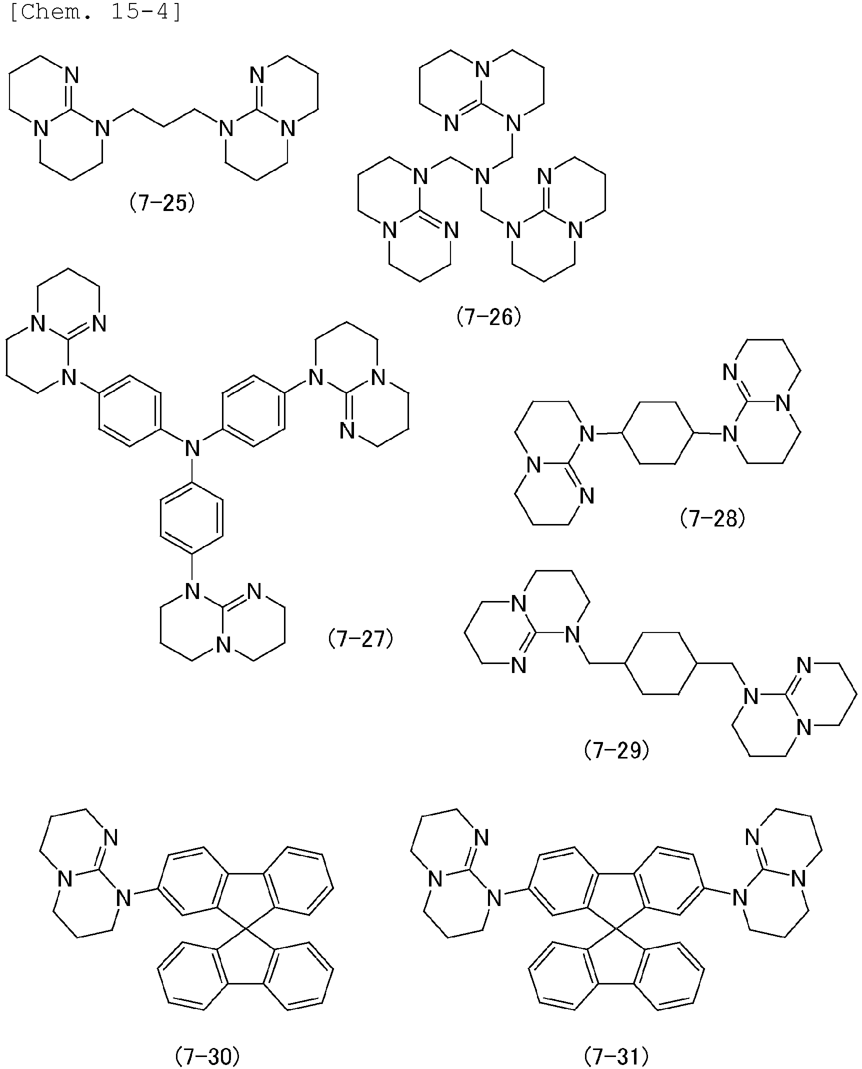

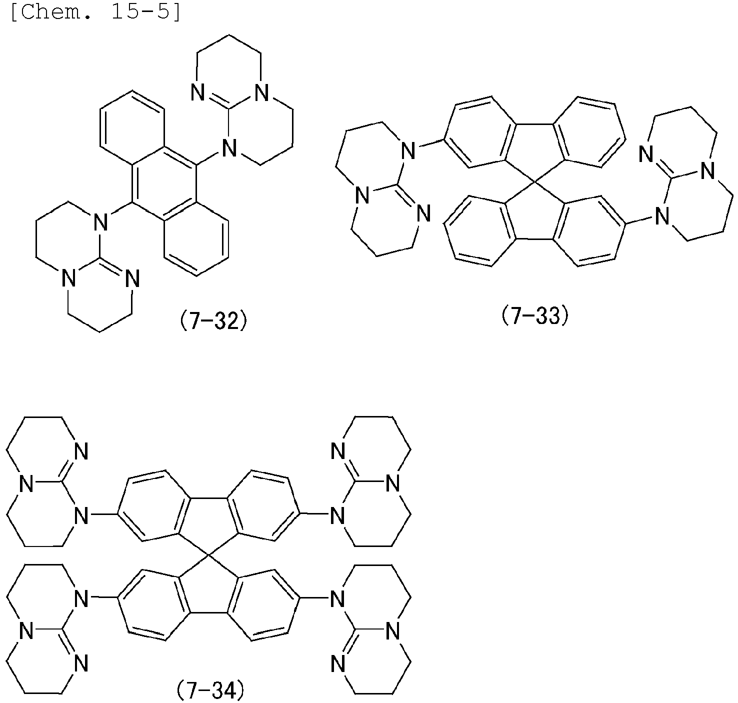

The compound represented by the formula (6) can be synthesized from an iodine-, bromine-, chlorine-, or fluorine-containing halogen compound and hexahydropyrimidopyrimidine as starting materials by the Ullmann coupling reaction, the Buchwald-Hartwig amination reaction, or the nucleophilic substitution reaction as shown in the following scheme (8).

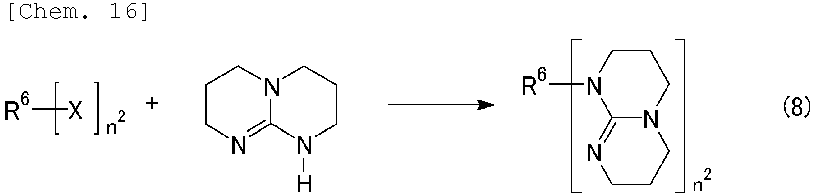

The material of the organic electron injection layer 6 may be any one that can be used as a ligand. Thus, the material of the organic electron injection layer 6 may be an acetylacetonate derivative containing oxygen atoms as coordinating elements as well as a compound containing nitrogen atoms.

The average thickness of the organic electron injection layer 6 is preferably 0.5 to 10 nm, more preferably 1 to 5 nm.

The average thickness of the electron injection layer 6 can be measured, for example, with a stylus profiler or a spectroscopic ellipsometer.

The organic electron injection layer may also be a layer mixed with the adjacent electron transport layer 7.

### "Electron transport material"

The material of the electron transport layer 7 may be any material that can be commonly used as a material of an electron transport layer, which may be used as needed.

Specific examples of the material of the electron transport layer 7 include phosphine oxide derivatives such as phenyl-dipyrenylphosphine oxide (POPy₂); pyridine derivatives such as tris-1,3,5-(3'-(pyridin-3"-yl)phenyl) benzene (TmPhPyB); quinoline derivatives such as (2-(3-(9-carbazolyl)phenyl)quinoline (mCQ)); pyrimidine derivatives such as 2-phenyl-4,6-bis(3,5-dipyridylphenyl) pyrimidine (BPyPPM); pyrazine derivatives; phenanthroline derivatives such as bathophenanthroline (BPhen); triazine derivatives such as 2,4-bis(4-biphenyl)-6-(4'-(2-pyridinyl)-4-biphenyl)-[1,3,5]triazine (MPT); triazole derivatives such as 3-phenyl-4-(1'-naphthyl)-5-phenyl-1,2,4-triazole (TAZ); oxazole derivatives; oxadiazole derivatives such as 2-(4-biphenyl)-5-(4-tert-butylphenyl-1,3,4-oxadiazole) (PBD); imidazole derivatives such as 2,2',2"-(1,3,5-benzenetriyl)-tris(1-phenyl-1-H-benzimidazole) (TPBI); aromatic carboxylic anhydrides such as naphthalene-1,4,5,8-tetracarboxylic dianhydride and 3,4,9,10-perylene tetracarboxylic dianhydride; aromatic imide compounds such as N,N'-dimethyl-3,4,9,10-perylene tetracarboxylic imide; carbonyl-containing compounds such as an isoindigo derivative, a 2,5-dihydropyrrolo[3,4-c]pyrrole-1,4-dione derivative (diketopyrrolopyrrole), and truxenone; 1,2,5-thiadiazole derivatives such as naphtho[1,2-c:5,6-c']bis[1,2,5] thiadiazole and benzo[c][1,2,5]thiadiazole; various metal complexes typified by bis[2-(2-hydroxyphenyl)benzothiazolato]zinc (Zn(BTZ)₂) and tris(8-hydroxyquinolinato)aluminum (Alq₃); organic silane derivatives typified by silole derivatives such as 2,5-bis(6'-(2',2"-bipyridyl))-1,1-dimethyl-3,4-diphenylsilole (PyPySPyPy); and boron-containing compounds such as tris(2,4,6-trimethyl-3-(pyridin-3-yl)phenyl)borane (3TPYMB) and compounds disclosed in Japanese Patent Application No. 2012-228460, Japanese Patent Application No. 2015-503053, Japanese Patent Application No. 2015-053872, Japanese Patent Application No. 2015-081108, and Japanese Patent Application No. 2015-081109. One or more of these may be used.

Examples of the material of the electron transport layer 7 include, in addition to these materials, aromatic hydrocarbon compounds which are a variety of hydrocarbon compounds each containing an aromatic ring, compounds having a nitrogen-boron bond, n-electron-rich heteroaromatic compounds each containing an aromatic ring such as a pyrrole ring, a furan ring, or a thiophene ring, and silole ring-containing compounds.

Particularly preferred among the materials of the electron transport layer 7 are phosphine oxide derivatives such as POPy₂, metal complexes such as Alq₃, and pyridine derivatives such as TmPhPyB.

When the organic EL device of the present invention includes a layer in which any of the materials of the organic electron injection layer 6 and any of the above-described electron transport materials are mixed, the electron transport layer 7 may be omitted. Also, a hole blocking layer may be formed from any of these materials as a hole blocking material. In addition, the hole blocking layer may be formed from a commonly used material, which may be used as needed.

The average thickness of the electron transport layer 7 is preferably 10 to 150 nm, more preferably 20 to 100 nm, but is not limited thereto.

The average thickness of the electron transport layer 7 can be measured with a stylus profiler or a spectroscopic ellipsometer.

### "Emitting layer"

The emitting layer 8 may be formed from any of the materials that can be commonly used for the emitting layer 8 or may be made of a mixture of these materials. For example, the emitting layer 8 may contain bis[2-[(2-benzothiazolyl)phenolato]zinc (II) (Zn(BTZ)₂) and tris[1-phenylisoquinoline]iridium(III) (Ir(piq)₃).

Examples of the material include various metal complexes such as a tridentate iridium complex having 2,2'-bipyridine-4,4'-dicarboxylic acid as a ligand, fac-tris(2-phenylpyridine)iridium (Ir(ppy)₃), 8-hydroxyquinoline aluminum (Alq₃), tris(4-methyl-8-quinolinolate) aluminum(III) (Almq₃), 8-hydroxyquinoline zinc (Znq₂), (1,10-phenanthroline)-tris-(4,4,4-trifluoro-1-(2-thienyl)-butane-1,3-dionate) europium(III) (Eu(TTA)₃(phen)), and 2,3,7,8,12,13,17,18-octaethyl-21H,23H-porphin platinum(II); benzene compounds such as distyrylbenzene (DSB) and diaminodistyrylbenzene (DADSB); naphthalene compounds such as naphthalene and Nile red; phenanthrene compounds such as phenanthrene; chrysene compounds such as chrysene and 6-nitrochrysene; perylene compounds such as perylene and N,N'-bis(2,5-di-t-butylphenyl)-3,4,9,10-perylene-di-carboxy imide (BPPC); coronene compounds such as coronene; anthracene compounds such as anthracene and bisstyrylanthracene; pyrene compounds such as pyrene; pyran compounds such as 4-(di-cyanomethylene)-2-methyl-6-(para-dimethylaminostyryl)-4H-pyran (DCM); acridine compounds such as acridine; stilbene compounds such as stilbene; thiophene compounds such as 2,5-dibenzooxazolethiophene; benzooxazole compounds such as benzooxazole; benzimidazole compounds such as benzimidazole; benzothiazole compounds such as 2,2'-(para-phenylenedivinylene)-bisbenzothiazole; butadiene compounds such as bistyryl(1,4-diphenyl-1,3-butadiene) and tetraphenylbutadiene; naphthalimide compounds such as naphthalimide; coumarin compounds such as coumarin; perynone compounds such as perynone; oxadiazole compounds such as oxadiazole; aldazine compounds; cyclopentadiene compounds such as 1,2,3,4,5-pentaphenyl-1,3-cyclopentadiene (PPCP); quinacridone compounds such as quinacridone and quinacridone red; pyridine compounds such as pyrrolopyridine and thiadiazolopyridine; spiro compounds such as 2,2',7,7'-tetraphenyl-9,9'-spirobifluorene; metallic or non-metallic phthalocyanine compounds such as phthalocyanine (H₂Pc) and copper phthalocyanine; and boron compound materials disclosed in JP 2009-155325 A, JP 2011-184430 A, and Japanese Patent Application No. 2011-6458.

The material of the emitting layer 8 may be a low-molecular compound or a high-molecular compound. The term "low-molecular material" as used herein refers to a material that is not a high-molecular material (polymer), and does not necessarily refer to a low molecular weight organic compound.

The average thickness of the emitting layer 8 is preferably 10 to 150 nm, more preferably 20 to 100 nm, but is not limited thereto.

The average thickness of the emitting layer 8 may be measured with a stylus profiler, or may be measured during formation of the emitting layer 8 with a quartz crystal thickness monitor.

### "Hole transport layer"

Examples of a hole transport organic material of the hole transport layer 9 include p-type high-molecular materials (organic polymers), p-type low-molecular materials, and combinations of these.

Specific examples of the material of the hole transport layer 9 include N,N'-di(1-naphthyl)-N,N'-diphenyl-1,1'-biphenyl-4,4'-diamine (α-NPD), N4,N4'-bis(dibenzo[b,d]thiophen-4-yl)-N4,N4'-diphenylbiphenyl-4,4'-diamine (DBTPB), polyarylamine, fluorene-arylamine copolymers, fluorene-bithiophene copolymers, poly(N-vinylcarbazole), polyvinylpyrene, polyvinylanthracene, polythiophene, polyalkylthiophenes, polyhexylthiophene, poly(p-phenylenevinylene), polythienylenevinylene, pyreneformaldehyde resin, ethylcarbazole formaldehyde resin, and derivatives of these. These materials of the hole transport layer 9 may be used as a mixture with other compounds. An example of a mixture containing polythiophene as the material of the hole transport layer 9 is poly(3,4-ethylenedioxythiophene/styrenesulfonate) (PEDOT/PSS).

The average thickness of the hole transport layer 9 is preferably 10 to 150 nm, more preferably 20 to 100 nm, but is not limited thereto.

The average thickness of the hole transport layer 9 can be measured with a stylus profiler or a spectroscopic ellipsometer, for example.

### "Hole injection layer"

The hole injection layer 10 may be formed from an inorganic material or an organic material. Since an inorganic material is more stable than an organic material, the use of an inorganic material easily imparts higher resistance to oxygen and water than the use of an organic material.

Non-limiting examples of the inorganic material include metal oxides such as vanadium oxide (V₂O₅), molybdenum oxide (MoO₃), and ruthenium oxide (RuO₂). Each of these may be used alone or two or more of these may be used in combination.

Examples of the organic material include a low-molecular material such as dipyrazino(2,3-f:2',3'-h]quinoxaline-2,3,6,7,10,11-hexacarbonitrile (HAT-CN) or 2,3,5,6-tetrafluoro-7,7,8,8-tetracyano-quinodimethane (F4-TCNQ) and poly(3,4-ethylenedioxythiophene)/polystyrene sulfonate (PEDOT:PSS).

The average thickness of the hole injection layer 10 is preferably 1 to 1000 nm, more preferably 5 to 50 nm, but is not limited thereto.

The average thickness of the hole injection layer 10 can be measured with a quartz crystal thickness monitor, a stylus profiler, or a spectroscopic ellipsometer during film formation.

### "Anode"

Examples of a material of the anode 11 include ITO, IZO, Au, Pt, Ag, Cu, Al, and alloys containing any of these. Preferred among these materials of the anode 11 are ITO, IZO, Au, Ag, and Al.

The average thickness of the anode 11 is preferably 10 to 1000 nm, more preferably 30 to 200 nm, even more preferably, 30 to 150 nm, but is not limited thereto. Even when the anode 11 is made of an opaque material, the anode 11 having an average thickness of about 10 to 30 nm can be used as a transparent anode for a top emission organic EL device.

The average thickness of the anode 11 can be measured with a quartz crystal thickness monitor during formation of the anode 11.

A mixture of any of these materials and Mg can be used as a cathode material when the organic EL device of the present invention has a conventional structure. In this case, the average thickness of the cathode is preferably the same as that of the anode 11.

### "Sealing"

The organic EL device 1 shown in FIG. 1 may be sealed, as needed.

For example, the organic EL device 1 shown in FIG. 1 may be sealed in a sealing container (not shown) having a recessed space for containing the organic EL device 1 by bonding the edge of the sealing container and the substrate 2 with an adhesive. Alternatively, the organic EL device 1 may be contained in a sealing container and sealed by filling the container with a sealing material made of an ultraviolet (UV) curable resin, for example. Also, for example, the organic EL device 1 shown in FIG. 1 may be sealed using sealing members including a plate member (not shown) placed on the anode 11 and a frame member (not shown) placed along an anode 11-side edge of the plate member, with the plate member being bound to the frame member and the frame member being bound to the substrate 2 with an adhesive.

When the organic EL device 1 is sealed using the sealing container or the sealing members, a desiccant to absorb moisture may be placed in the sealing container or in the sealing members. Also, the sealing container or the sealing members may be made of a moisture absorbing material. There may be a space in the sealing container or in the sealing members after sealing.

Examples of a material of the sealing container or the sealing members used to seal the organic EL device 1 shown in FIG. 1 include a resin material and a glass material. Examples of the resin material and glass material of the sealing container or sealing members include those mentioned as the material of the substrate 2.

The organic EL device 1 according to the first aspect of the present invention has higher durability when having a structure in which the cathode is formed from ITO, the metal electron injection layer 5 is formed from a metal thin film having a work function of 4.0 eV or higher, and the organic electron injection layer 6 is formed from an organic material having a coordination ability represented by the formula (1) than when having a structure in which the electron injection layer is formed from an alkali metal, which is unstable in the atmosphere, for example.

The organic EL device 1 according to the second aspect of the present invention has higher durability when having a structure in which the organic electron injection layer 5 is formed from an organic material having a coordination ability represented by the formula (1) than when having a structure in which the electron injection layer is formed only from an alkali metal, which is unstable in the atmosphere, for example.

Thus, the sealing container or sealing members are required to have a water vapor transmission rate of about 10⁻⁴ to 10⁻³ g/m²/day for sufficient prevention of degradation of the organic EL device 1. A resin material having a water vapor transmission rate of about 10⁻³ g/m²/day or less can be used as the material of the sealing container or sealing members, and thereby the organic EL device 1 with excellent flexibility can be obtained.

### "Method for producing organic EL device"

Next, a method for producing an organic EL device 1 having an inverted structure according to the first aspect of the present invention shown in FIG. 1 will be described as an example of the method for producing an organic EL device of the present invention. The organic EL device of the present invention may have a conventional structure. The organic EL device having a conventional structure is produced, for example, only by reversing the structure so that the position of the anode and the position of the hole injection layer are reversed and may be produced by any film forming method.

In order to produce the organic EL device 1 shown in FIG. 1, first, a cathode 3 is formed on a substrate 2.

The cathode 3 may be formed by, for example, a sputtering method, a vacuum deposition method, a sol-gel method, a spray pyrolysis deposition (SPD) method, an atomic layer deposition (ALD) method, a vapor deposition method, or a liquid phase deposition method. The cathode 3 may be formed by bonding metal foil.

In the case of the organic EL device according to the first aspect of the present invention, a metal electron injection layer 5 is then formed on the cathode 3. The metal electron injection layer 5 is formed, for example, by a method such as vacuum deposition, and then an organic electron injection layer 6 is formed on the metal electron injection layer 5.

An example of the organic EL device according to the second aspect of the present invention is shown in FIG. 7. In the organic EL device according to the second aspect of the present invention, an organic electron injection layer 6 is formed on a cathode 3.

The organic electron injection layer 6 is formed by a method such as vacuum deposition, for example.

Next, an electron transport layer 7, an emitting layer 8, and a hole transport layer 9 are formed on the organic electron injection layer 6 in the stated order.

The electron transport layer 7, the emitting layer 8, and the hole transport layer 9 may be formed by any method, and may be formed by appropriate methods selected from known various forming methods depending on the properties of the materials of the electron transport layer 7, the emitting layer 8, and the hole transport layer 9.

Next, a hole injection layer 10 is formed on the hole transport layer 9, and an anode 11 is formed on the layer 10. These layers may also be formed by appropriate methods selected from known various forming methods depending on the properties of the materials of the layers.

The organic EL devices 1 shown in FIG. 1 and FIG. 7 are obtained through the above steps.

### "Sealing method"

The organic EL device 1 of the present invention can be sealed using a method typically used for sealing organic EL devices.

### EXAMPLES

The present invention is described in more detail with reference to examples below, but the present invention is not limited to these examples. Herein, "%" means "mol%" unless otherwise stated.

### (Synthesis Example 1)

A compound represented by the following formula (9) was synthesized by the method described below.

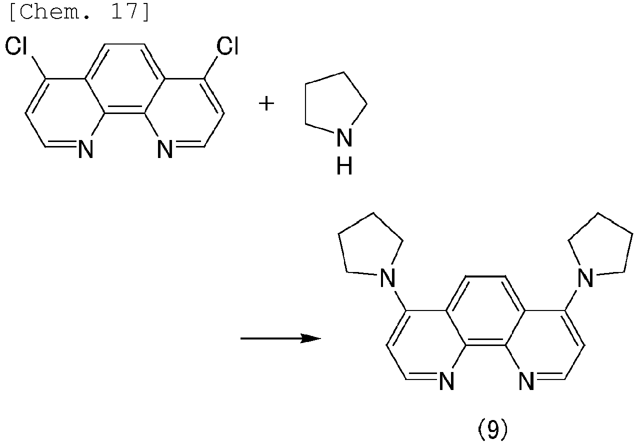

A mixture of 4,7-dichloro-1,10-phenanthroline (3.00 g) and pyrrolidine (19.5 mL) in a 100-mL recovery flask was heated in an oil bath at 100°C to reflux for one hour. The mixture was cooled to room temperature and concentrated under reduced pressure. Water was added to the concentrate, and they were subjected to ultrasonication to precipitate solids, which were collected by filtration. The resulting solids were dried under reduced pressure and dissolved in methanol (100 mL). To the mixture was added activated carbon, the contents were stirred at room temperature for one hour, and insoluble matters were separated by filtration. The filtrate was concentrated under reduced pressure to obtain solids, which were recrystallized with methanol (9 mL). The resulting solids were washed with a small amount of methanol and dried under reduced pressure. Thus, the compound represented by the formula (9) was obtained as a white solid (1.69 g, 44%).

### (Synthesis Example 2)

A compound represented by the following formula (7-2) was synthesized by the following method.

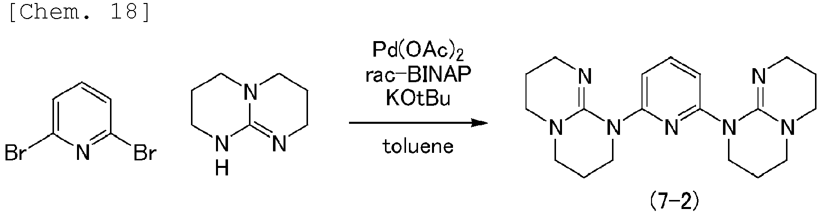

A 200-mL three-necked flask was charged with rac-BINAP (747 mg) and toluene (67 mL), which were heated in a nitrogen atmosphere at 90°C for dissolution. The mixture was cooled to room temperature, palladium acetate (180 mg) was added thereto, and the contents were stirred at room temperature for one hour. To the mixture were added 2,6-dibromopyridine (4.74 g), 1,3,4,6,7,8-hexahydro-2H-pyrimido[1,2-a]pyrimidine (6.13 g), and KOtBu (6.28 g), and the contents were heated with stirring at 90°C overnight. The mixture was cooled to room temperature, diethyl ether was added thereto to precipitate solids, which were separated by filtration, and the filtrate was concentrated. To the resulting residue was added acetone to precipitate solids, which were collected by filtration. Thus, the compound represented by the formula (7-2) (3.7 g, 52.5%) was obtained.

### <Organic electroluminescence device according to first aspect of the present invention>

### (Production of organic electroluminescence device)

### (Example 1)

(Step 1) A glass substrate 2 with an ITO electrode layer (cathode 3) (thickness 0.7 mm) was prepared. This substrate was subjected to UV/ozone cleaning for 20 minutes.

(Step 2) Next, the substrate subjected to the treatment in (Step 1) was placed in a vacuum apparatus, the pressure was reduced to 5 × 10⁻⁵ Pa or lower, and a 10-nm thick silver layer and a 1-nm thick zinc layer were formed respectively as a second cathode 3 and a metal electron injection layer 5 by vacuum deposition.

(Step 3) Next, the following layers were stacked by vacuum deposition: a 15-nm organic electron injection layer 6 formed by co-evaporation of an organic electron injection layer material represented by the formula (9) and an electron transport material KHLHEI-02 available from Chemipro Kasei Kaisha, Ltd.; a 10-nm thick hole blocking layer 12 made of KHLHS-04 available from Chemipro Kasei Kaisha, Ltd.; a 25-nm thick emitting layer 8 formed by co-evaporation of KHLHS-03, KHLHS-04, and KHLDG-01 each available from Chemipro Kasei Kaisha, Ltd.; and a 40-nm thick hole transport layer 9 made of KHLHS-03 available from Chemipro Kasei Kaisha, Ltd.

(Step 4) Next, a hole injection layer 10 was formed on the hole transport layer 9. Specifically, a 10-nm thick molybdenum oxide film was formed by vacuum deposition, which was vapor deposition.

(Step 5) Finally, an anode 11 was formed on the hole injection layer 10. Specifically, a 100-nm thick aluminum film was formed by vacuum deposition.

Through (Step 1) to (Step 5), an organic electroluminescence device having a structure shown in FIG. 2 was produced.

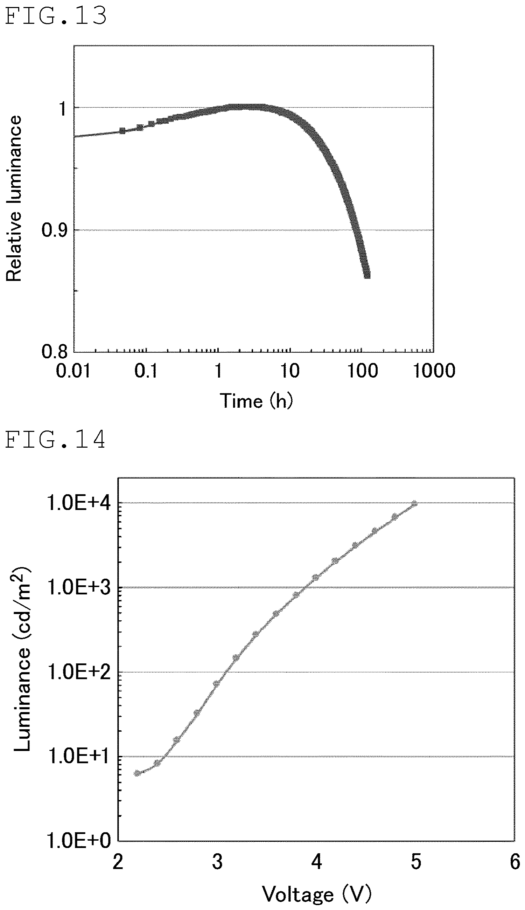

(Step 6) The characteristics (voltage-luminance characteristics and lifetime characteristics) of the organic electroluminescence device were evaluated according to the following <Measurement of luminescence characteristics of organic electroluminescence device>. The results are shown in FIGS. 12 and 13.

### <Measurement of luminescence characteristics of organic electroluminescence device>

"Model 2400 SourceMeter" available from Keithley Instruments was used to apply a voltage to the device and to measure the current. The luminance was measured with "BM-7" available from Topcon Corporation. The measurement was performed under nitrogen.

### <Measurement of lifetime of organic electroluminescence device>

The luminance degradation was measured under a constant current in a nitrogen atmosphere.

### (Example 2)

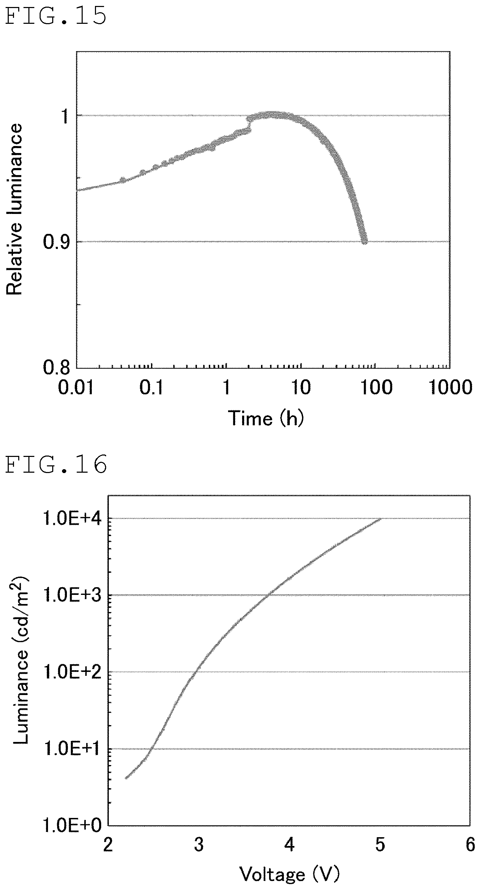

An organic electroluminescence device was produced as in Example 1, except that a 1-nm thick copper layer was formed instead of the zinc layer in (Step 2). As (Step 6), the characteristics of the organic electroluminescence device were evaluated as in Example 1. The results are shown in FIGS. 14 and 15.

### (Example 3)

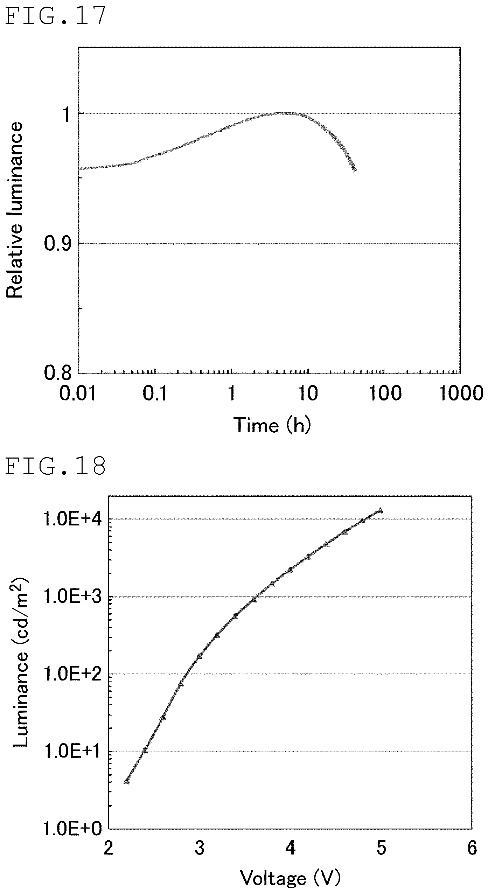

An organic electroluminescence device was produced as in Example 1, except that a 1-nm thick aluminum layer was formed instead of the zinc layer in (Step 2). As (Step 6), the characteristics of the organic electroluminescence device were evaluated as in Example 1. The results are shown in FIGS. 16 and 17.

### (Example 4)

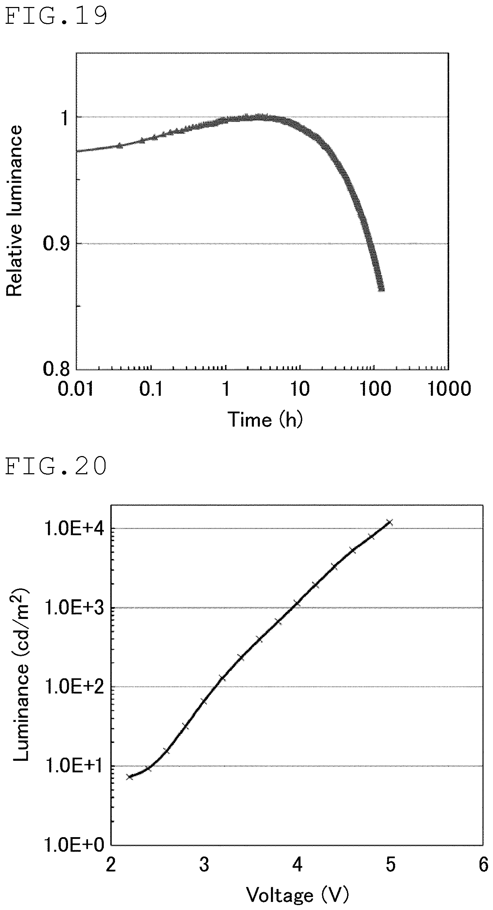

An organic electroluminescence device was produced as in Example 1, except that (Step 2) was replaced by (Step 2-5) below. As (Step 6), the characteristics of the organic electroluminescence device were evaluated as in Example 1. The results are shown in FIGS. 18 and 19.

(Step 2-5) Next, the substrate subjected to the treatment in (Step 1) was placed in a vacuum apparatus, the pressure was reduced to 5 × 10⁻⁵ Pa or lower, and a 11-nm thick film was prepared by co-evaporation under vacuum of silver which was the material of the second cathode and zinc as a metal electron injection layer 4 containing a mixture of silver and zinc.

### (Comparative Example 1)

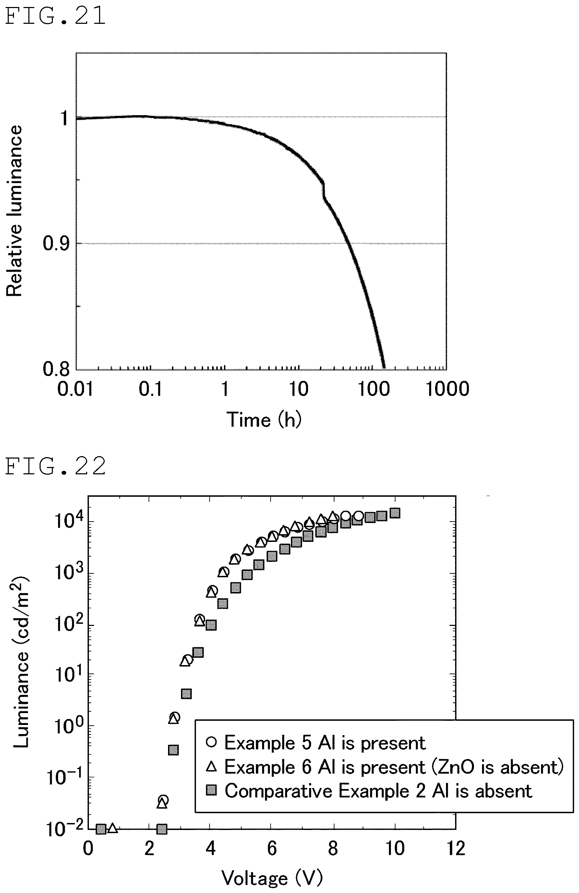

An organic electroluminescence device was produced as in Example 1, except that (Step 2) was replaced by (Step 2-51) below. As (Step 6), the characteristics of the organic electroluminescence device were evaluated as in Example 1. The results are shown in FIGS. 20 and 21.

(Step 2-51) This substrate was fixed to a substrate holder of a mirrortron sputtering apparatus having zinc metal targets. The pressure was reduced to about 5 × 10⁻⁵ Pa, and then sputtering was performed under introduced argon and oxygen to form a zinc oxide layer (a metal oxide layer) having a thickness of about 2 nm as a substitute for the metal electron injection layer 5.

Comparison of the devices of Examples 1 to 4 to the device of Comparative Example 1 including the metal oxide layer 4, but not including the metal electron injection layer 5, shows an effect that the luminance under the same voltage is higher and the lifetime is longer in the devices of Examples 1 to 4.

### (Example 5)

(Step 1) A commercially available transparent glass substrate having an average thickness of 0.7 mm with a 150-nm thick electrode (cathode 3) made of ITO with a pattern of 3 mm width was prepared as a substrate 2. The substrate 2 with the cathode 3 was ultrasonically cleaned in acetone and isopropanol each for 10 minutes and then boiled in isopropanol for five minutes. Thereafter, the substrate 2 with the cathode 3 was taken out from isopropanol, dried by blowing nitrogen, and subjected to UV/ozone cleaning for 20 minutes.

(Step 2) The substrate 2 with the cathode 3 cleaned in (Step 1) was fixed to a substrate holder of a mirrortron sputtering apparatus having zinc metal targets. The pressure in the chamber of the sputtering apparatus was reduced to about 1 × 10⁻⁴ Pa, and sputtering was performed under introduced argon and oxygen to form a zinc oxide layer (metal oxide layer 4) having a thickness of about 3 nm on the cathode 3 of the substrate 2. Upon the formation of the zinc oxide layer, no zinc oxide film was formed on part of the ITO electrode (cathode 3) in order to lead out the electrode. The substrate 2 with the metal oxide layer 4 was annealed in the atmosphere at 400°C for one hour.

(Step 3) A 2-nm thick aluminum film was formed as a metal electron injection layer 5 by vacuum deposition using resistance heating.

(Step 4) After forming the metal electron injection layer 5, a 1-nm thick organic electron injection layer 6 made of the compound represented by the formula (7-2) was formed by vacuum deposition.

(Step 5) Subsequently, a 10-nm thick electron transport layer 7 made of bis[2-(2-benzothiazolyl)phenolato]zinc(II) (Zn(BTZ)₂) represented by the formula (10) below was formed by vacuum deposition. Subsequently, a 20-nm thick emitting layer 8 was formed by co-evaporation of Zn(BTZ)₂ as a host and tris[1-phenylisoquinoline]iridium(III) (Ir(piq)₃) represented by the formula (11) below as a dopant. The doping concentration was controlled such that Ir(piq)₃ was 6% by mass relative to the entire emitting layer 8. Next, a 50-nm thick hole transport layer 9 made of N,N'-di(1-naphthyl)-N,N'-diphenyl-1,1'-biphenyl-4,4'-diamine (α-NPD) represented by the formula (12) below was formed, on the substrate 2 with the emitting layer 8 and the previous layers. Further, a 10-nm thick hole injection layer 10 made of 1,4,5,8,9,12-hexaazatriphenylene-2,3,6,7,10,11-hexacarbonitrile (HAT-CN) represented by the formula (13) below was formed. Next, a 100-nm thick aluminum film was formed, as an anode 11, on the substrate 2 with the hole injection layer 10 and the previous layers by vacuum deposition.

The anode 11 was formed using a stainless steel evaporation mask to have a 3-mm-width-band-like evaporation area. The produced organic EL device had an emitting area of 9 mm².

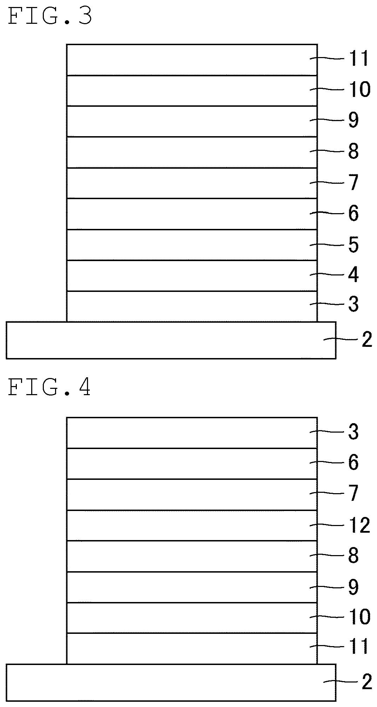

(Step 6) Next, the substrate 2 with the anode 11 and the previous layers was placed in a glass cap (sealing container) with a recessed space, and the container was filled with a sealing material made of an ultraviolet (UV) curable resin to seal the substrate. Thus, an organic electroluminescence device having a structure shown in FIG. 3 was produced.

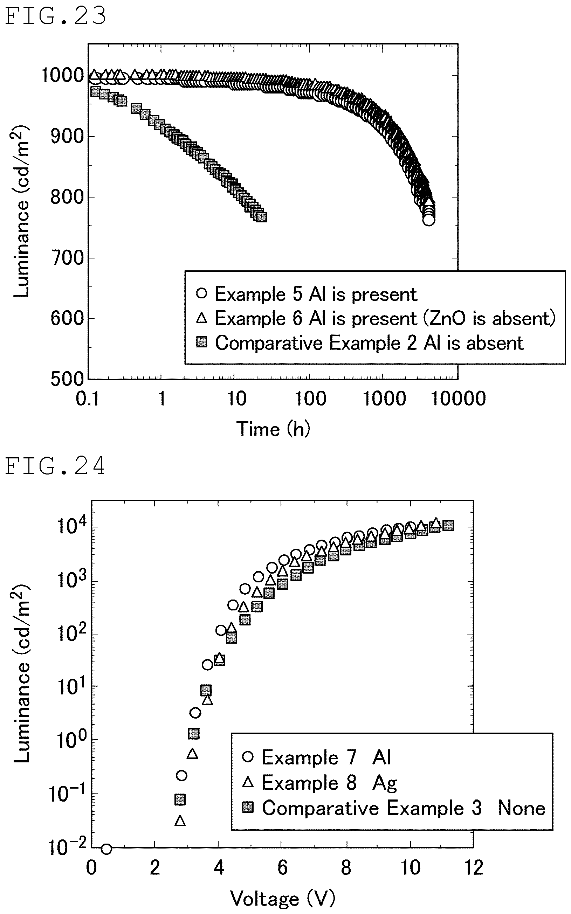

(Step 7) The characteristics (voltage-luminance characteristics and lifetime characteristics) of the organic electroluminescence device were measured according to the following <Measurement of luminescence characteristics of organic electroluminescence device>. The results are shown in FIGS. 22 and 23.

### <Measurement of luminescence characteristics of organic electroluminescence device>

To the device was applied a voltage using "Model 2400 SourceMeter" available from Keithley Instruments, and the luminance was measured using "LS-100" available from Konica Minolta, Inc. Thus, the relationship between the applied voltage and the luminance was determined.

### <Measurement of lifetime of organic electroluminescence device>

Using an "organic EL lifetime meter" available from EHC, the relationship between the elapsed time from the start of driving with a constant current and the relative luminance was examined. Specifically, the voltage was automatically adjusted so that a constant current flowed through the organic electroluminescence device, and the relative luminance was measured with respect to the elapsed time from the start of driving with the constant current using a luminance meter (LS-110) available from Konica Minolta Japan, Inc. The current value was set for each organic electroluminescence device so that the luminance at the start of measurement was 1000 cd/m².

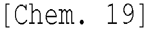

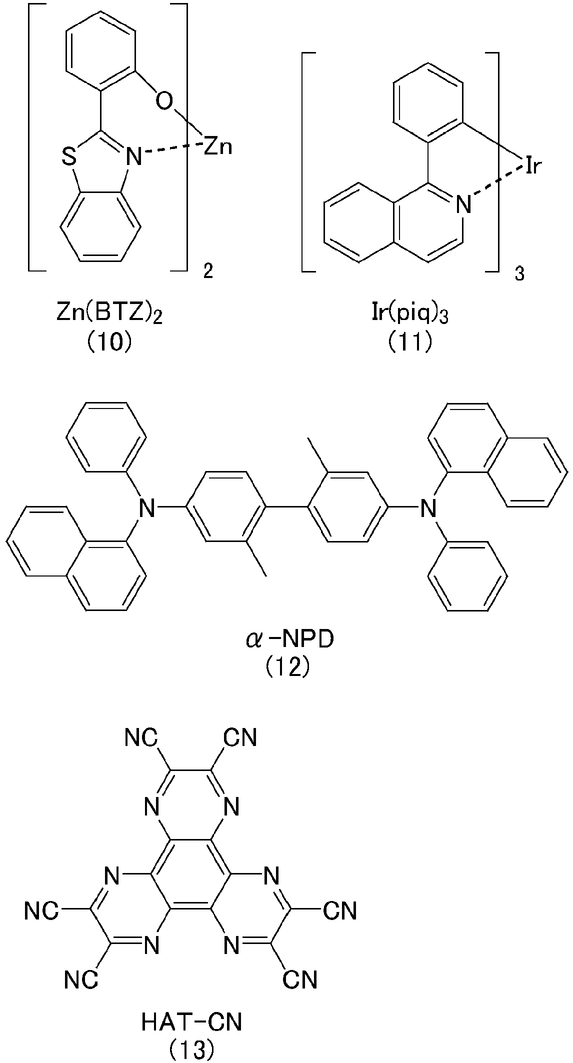

### (Example 6)

An organic electroluminescence device of Example 6 was produced as in Example 5, except that (Step 2) was omitted not to form the metal oxide layer 4. As (Step 7), the characteristics of the organic electroluminescence device were evaluated as in Example 5. The results are shown in FIGS. 22 and 23.

### (Comparative Example 2)

An organic electroluminescence device of Comparative Example 2 was produced as in Example 5, except that (Step 3) was omitted not to form the metal electron injection layer 5. As (Step 7), the characteristics of the organic electroluminescence device were evaluated as in Example 5. The results are shown in FIGS. 22 and 23.

As can be seen from FIG. 22, the devices of Examples 5 and 6 exhibit higher luminance than the device of Comparative Example 2 including no metal electron injection layer under the same voltage. In addition, as can be seen from FIG. 23, the devices of Examples 5 and 6 have better results of the lifetime characteristics than the device of the comparative example. These results demonstrate that the effects of the present invention are clearly achieved even in the case of using an organic electron injection layer including the hexahydropyrimidopyrimidine compound represented by the formula (7-2). As shown in Examples 5 and 6, the metal electron injection layer 5 was found to be effective both on the ITO cathode and on the metal oxide layer such as zinc oxide.

### (Example 7)

An organic electroluminescence device of Example 7 was produced as in Example 5, except that (Step 2) was omitted not to form the metal oxide layer 4 and an organic electron injection layer 5 was formed from the material represented by the formula (9) in (Step 4) without using the compound represented by the formula (7-2). As (Step 7), the voltage-luminance characteristics of the organic electroluminescence device were evaluated as in Example 5. The results are shown in FIG. 24.

### (Example 8)

An organic electroluminescence device of Example 8 was produced as in Example 5, except that (Step 2) was omitted not to form the metal oxide layer 4, a metal electron injection layer 5 was formed from silver in (Step 3) without using aluminum, and an organic electron injection layer 6 was formed from the material represented by the formula (9) in (Step 4) without using the compound represented by the formula (7-2). As (Step 7), the voltage-luminance characteristics of the organic electroluminescence device were evaluated as in Example 5. The results are shown in FIG. 24.

### (Comparative Example 3)

An organic electroluminescence device of Comparative Example 3 was produced as in Example 5, except that (Step 3) was omitted not to form the metal electron injection layer 5 and an organic electron injection layer 6 was formed from the material represented by the formula (9) in (Step 4) without using the compound represented by the formula (7-2). As (Step 7), the voltage-luminance characteristics of the organic electroluminescence device were evaluated as in Example 5. The results are shown in FIG. 24.

The devices of Examples 7 and 8 exhibit higher luminance than the device of Comparative Example 3 including an ITO cathode, but not including the metal electron injection layer 5, under the same voltage, indicating that the metal electron injection layer 5 is effective even on ITO.

### (Example 9)

(Step 1) A substrate 2 with an ITO electrode layer (anode 11) (0.7 mm thick) was prepared. This substrate was subjected to UV/ozone cleaning for 20 minutes.

(Step 2) The substrate subjected to the treatment in (Step 1) was placed in a vacuum apparatus, and the pressure was reduced to 5 × 10⁻⁵ Pa or lower.

(Step 3) Next, a hole injection layer 10 was formed. Specifically, a 10-nm thick molybdenum oxide film was formed by vacuum deposition, which was vapor deposition.

(Step 4) Next, the following layers were stacked by vacuum deposition: a 25-nm thick hole transport layer 9 made of KHLHS-03 available from Chemipro Kasei Kaisha, Ltd.; a 35-nm thick emitting layer 8 formed by co-evaporation of KHLHS-03, KHLHS-04, and KHLDG-01 each available from Chemipro Kasei Kaisha, Ltd.; a 15-nm thick hole blocking layer 12 made of KHLHS-04 available from Chemipro Kasei Kaisha, Ltd.; and a 15-nm thick electron transport layer 7 made of KHLHEI-02 available from Chemipro Kasei Kaisha, Ltd.

(Step 5) Next, a 1.5-nm thick organic electron injection layer 6 made the material represented by the formula (9) was formed by vacuum deposition. Finally, a cathode 3 was formed. Specifically, a 100-nm thick aluminum film was formed by vacuum deposition.

Through (Step 1) to (Step 5), an organic electroluminescence device having a structure shown in FIG. 4 was produced.

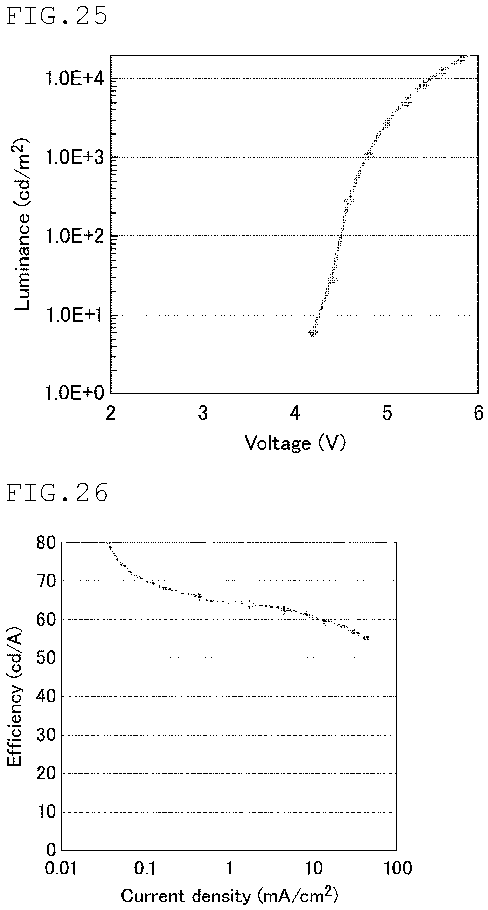

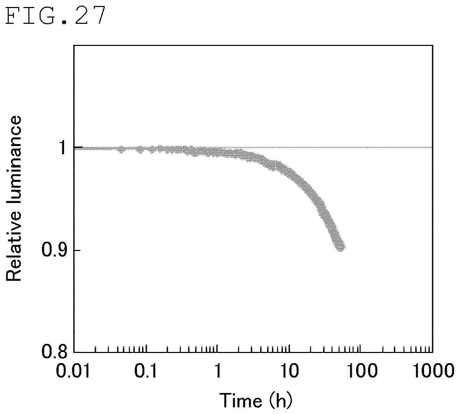

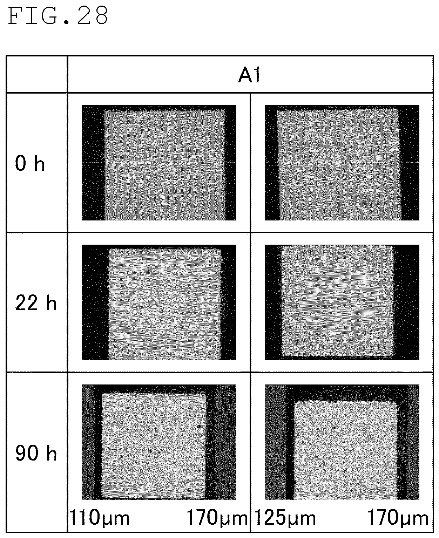

(Step 6) The characteristics (voltage-luminance characteristics, current density-efficiency characteristics, and lifetime characteristics) of the organic electroluminescence device were evaluated according to <Measurement of luminescence characteristics of organic electroluminescence device> and <Measurement of lifetime of organic electroluminescence device> described below. The results are shown in FIGS. 25 to 27. Furthermore, the device was allowed to stand in the atmosphere without sealing in order to examine stability in the atmosphere, and the luminescence was observed at the initial stage, after 22 hours, and after 90 hours. The observation results are shown in FIG. 28. The numerical values described in the observation results after 90 hours in FIG. 28 each indicate the distance between the edge of the emitting surface at 0 hours and the edge of the eroded emitting surface after 90 hours.

### <Measurement of luminescence characteristics of organic electroluminescence device>

"Model 2400 SourceMeter" available from Keithley Instruments was used to apply a voltage to the device and to measure the current. The luminance was measured with "BM-7" available from Topcon Corporation. The measurement was performed under nitrogen.

### <Measurement of lifetime of organic electroluminescence device>

The luminance degradation was measured under a constant current in a nitrogen atmosphere.

### (Example 10)

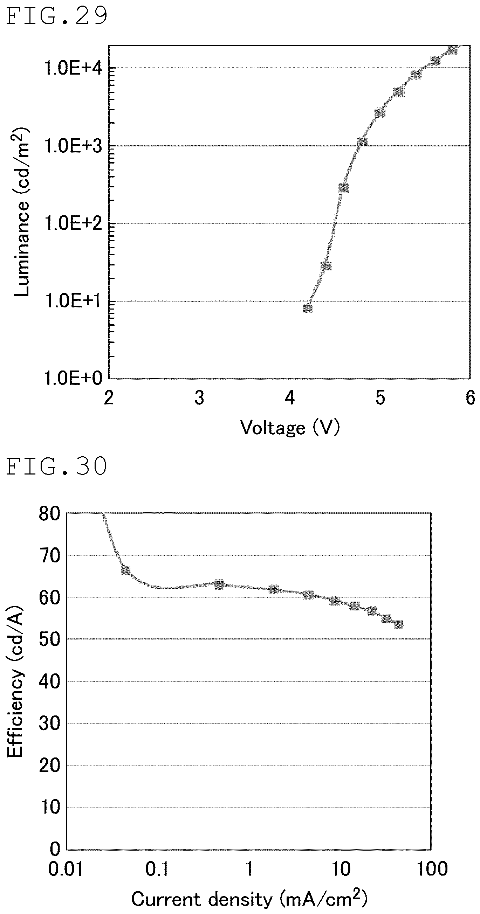

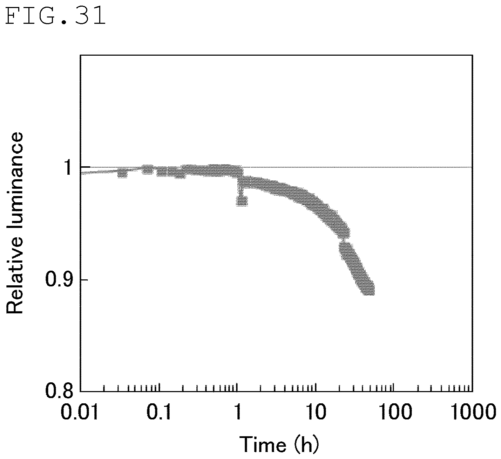

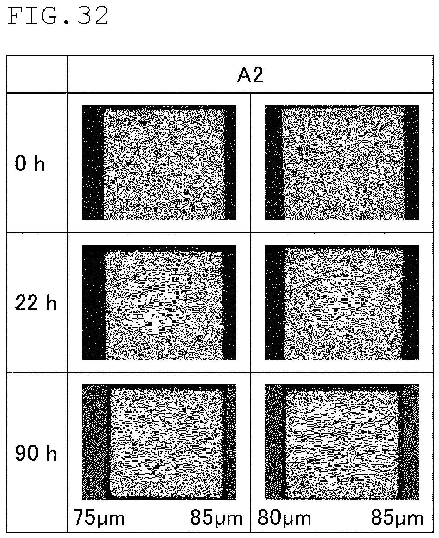

An organic electroluminescence device was produced as in Example 9, except that (Step 5) was replaced by (Step 5-A2) below. As (Step 6), the characteristics of the organic electroluminescence device were evaluated as in Example 9. The results are shown in FIGS. 29 to 31. Furthermore, the device was allowed to stand in the atmosphere without sealing in order to examine stability in the atmosphere, and the luminescence was observed at the initial stage, after 22 hours, and after 90 hours. The observation results are shown in FIG. 32. The numerical values described in the observation results after 90 hours in FIG. 32 each indicate the distance between the edge of the emitting surface at 0 hours and the edge of the eroded emitting surface after 90 hours.

(Step 5-A2) Next, a 1.5-nm thick organic electron injection layer 6 made of the material represented by the formula (9) was formed, and a 1-nm thick zinc film was formed as a metal electron injection layer 5 by vacuum deposition. Finally, a cathode 3 was formed. Specifically, a 100-nm thick aluminum film was formed by vacuum deposition.

### (Comparative Example 4)

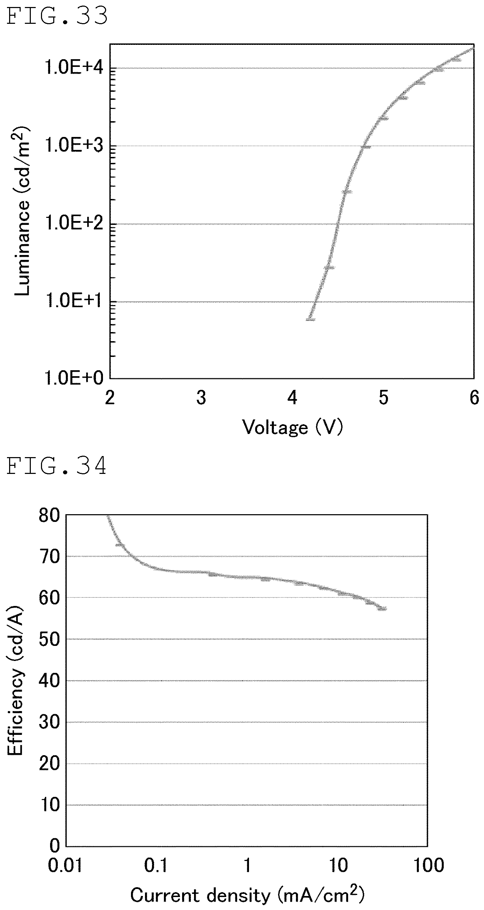

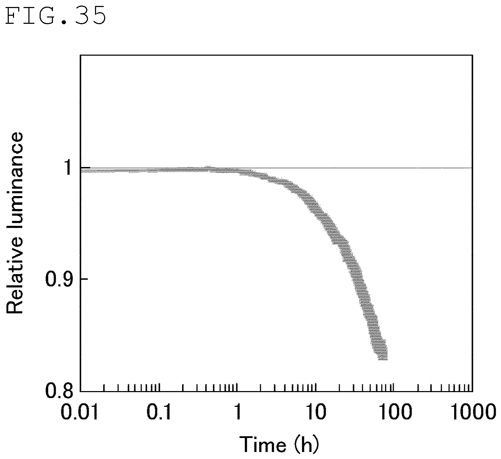

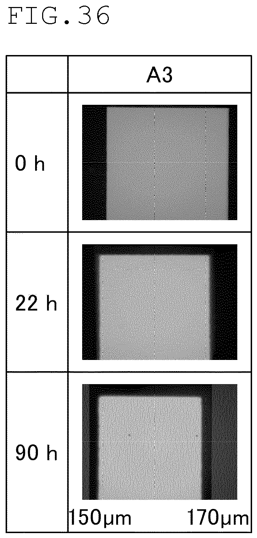

An organic electroluminescence device was produced as in Example 9, except that (Step 5) was replaced by (Step 5-A3) below. As (Step 6), the characteristics of the organic electroluminescence device were evaluated as in Example 9. The results are shown in FIGS. 33 to 35. Furthermore, the device was allowed to stand in the atmosphere without sealing in order to examine stability in the atmosphere, and the luminescence was observed at the initial stage, after 22 hours, and after 90 hours. The observation results are shown in FIG. 36. The numerical values described in the observation results after 90 hours in FIG. 36 each indicate the distance between the edge of the emitting surface at 0 hours and the edge of the eroded emitting surface after 90 hours.

(Step 5-A3) Next, instead of the organic electron injection layer 6, a 1-nm thick LiF film was formed by vacuum deposition. Finally, a cathode 3 was formed. Specifically, a 100-nm thick aluminum film was formed by vacuum deposition.

### (Example 11)

(Step 1) A substrate 2 with an ITO electrode layer (cathode 3) (0.7 mm thick) was prepared. This substrate was subjected to UV/ozone cleaning for 20 minutes.

(Step 2) Next, the substrate subjected to the treatment in (Step 1) was placed in a vacuum apparatus, the pressure was reduced to 5 × 10⁻⁵ Pa or lower, and a layer having a total thickness of 14.85 nm containing silver and zinc in a volume ratio of 10:1 was formed thereon as a layer serving as a second cathode and a metal electron injection layer 5 by vacuum deposition.

(Step 3) Next, the following layers were stacked by vacuum deposition: a 3-nm thick organic electron injection layer 6 made of an organic electron injection layer material represented by the formula (9); a 15-nm thick electron transport layer 7 made of an electron transport material KHLHEI-02 available from Chemipro Kasei Kaisha, Ltd.; a 15-nm thick hole blocking layer 12 made of KHLHS-04 available from Chemipro Kasei Kaisha, Ltd.; a 35-nm thick emitting layer 8 formed by co-evaporation of KHLHS-03, KHLHS-04, and KHLDG-01 each available from Chemipro Kasei Kaisha, Ltd.; and a 25-nm thick hole transport layer 9 made of KHLHS-03 available from Chemipro Kasei Kaisha, Ltd.

(Step 4) Next, a hole injection layer 10 was formed on the hole transport layer 9. Specifically, a 10-nm thick molybdenum oxide film was formed by vacuum deposition, which was vapor deposition.

(Step 5) Finally, an anode 11 was formed on the hole injection layer 10. Specifically, a 100-nm thick aluminum film was formed by vacuum deposition.

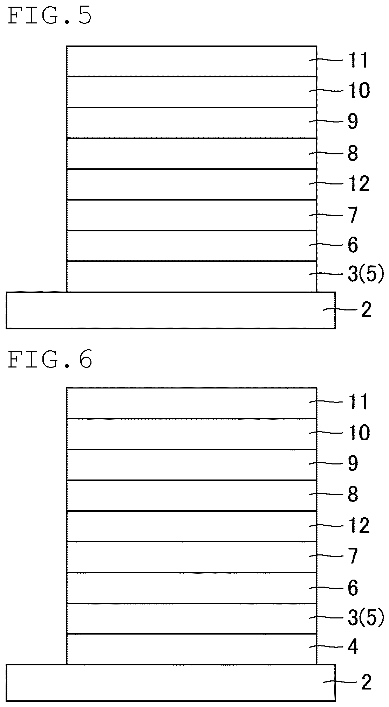

Through (Step 1) to (Step 5), an organic electroluminescence device having a structure shown in FIG. 5 was produced.

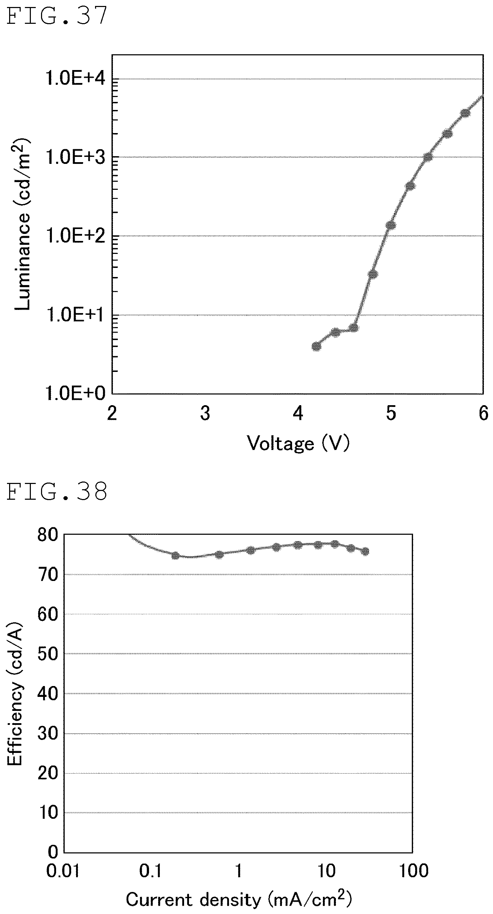

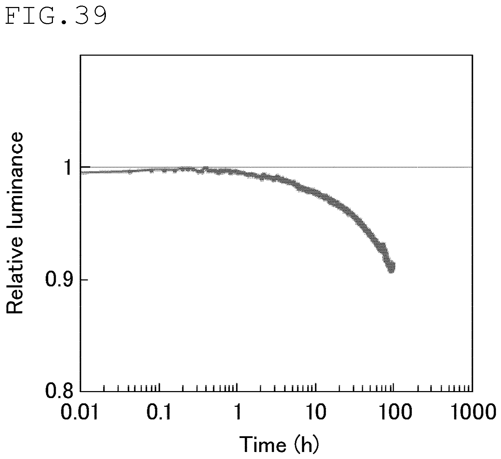

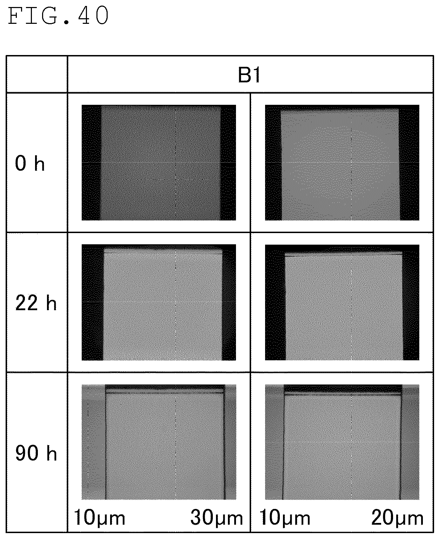

(Step 6) The characteristics (voltage-luminance characteristics, current density-efficiency characteristics, and lifetime characteristics) of the organic electroluminescence device were evaluated as in Example 9. The results are shown in FIGS. 37 to 39. Furthermore, the device was allowed to stand in the atmosphere without sealing in order to examine stability in the atmosphere, and the luminescence was observed at the initial stage, after 22 hours, and after 90 hours. The observation results are shown in FIG. 40. The numerical values described in the observation results after 90 hours in FIG. 40 each indicate the distance between the edge of the emitting surface at 0 hours and the edge of the eroded emitting surface after 90 hours.

The results of Examples 9 to 11 and Comparative Example 4 reveal the following.

The device of Comparative Example 4 includes the same light-emitting material and the like as those in Example 9 and has a conventional structure, which is a common structure. The devices of Examples 9 and 10 have a conventional structure but include an electron injection layer made of the material(s) in the present invention, i.e., include a metal electron injection layer made of Al or Zn. The device of Example 11 has an inverted structure and uses Ag and Zn as materials for a layer serving as a cathode and a metal electron injection layer. The efficiency of the device of Example 11 is slightly higher than those of the other devices, but is not so much different therefrom. In terms of operational lifetime, compared to the comparative example, the time required for luminance to decrease by 10%, for example, is longer in the order of Comparative Example 4 < Example 10 < Example 9 < Example 11. In particular, the time in Example 11 is 3 to 4 times longer than that in the comparative example. In terms of stability in the atmosphere, the area of non-emitting portions (eroded portions) is smaller in the order of Comparative Example 4 > Example 10 > Example 9 > Example 11, indicating that the device of Example 11 is stable in the atmosphere.

### (Example 12)

(Step 1) A resist material was applied to a barrier film (PT7/25GT3: thickness 25 um, WVTR: 4 × 10⁻³ g/m²/day) available from OIKE & Co., Ltd. to form a film, and a 24-nm thick zinc oxide film was formed with a sputtering device as a metal oxide layer 4.

(Step 2) Next, the substrate subjected to the treatment in (Step 1) was placed in a vacuum apparatus, the pressure was reduced to 5 × 10⁻⁵ Pa or lower, and a layer having a total thickness of 14.85 nm containing silver and zinc in a volume ratio of 10:1 was formed thereon as a layer serving as a cathode 3 and a metal electron injection layer 5 by vacuum deposition.

(Step 3) Next, the following layers were stacked by vacuum deposition: a 3-nm thick organic electron injection layer 6 made of an organic electron injection layer material represented by the formula (9); a 15-nm thick electron transport layer 7 made of an electron transport material KHLHEI-02 available from Chemipro Kasei Kaisha, Ltd.; a 15-nm thick hole blocking layer 12 made of KHLHS-04 available from Chemipro Kasei Kaisha, Ltd.; a 35-nm thick emitting layer 8 formed by co-evaporation of KHLHS-03, KHLHS-04, and KHLDG-01 each available from Chemipro Kasei Kaisha, Ltd.; and a 25-nm thick hole transport layer 9 made of KHLHS-03 available from Chemipro Kasei Kaisha, Ltd.

(Step 4) Next, a hole injection layer 10 was formed on the hole transport layer 9. Specifically, a 10-nm thick molybdenum oxide film was formed by vacuum deposition, which was vapor deposition.

(Step 5) Finally, an anode 11 was formed on the hole injection layer 10. Specifically, a 200-nm thick aluminum film was formed by vacuum deposition. The workpiece was sealed with a barrier film (thickness: 25 um, WVTR: 4 × 10⁻³ g/m²/day) available from OIKE & Co., Ltd.

Through (Step 1) to (Step 5), a film organic electroluminescence device having a structure shown in FIG. 6 was produced. The thickness was about 98 um.

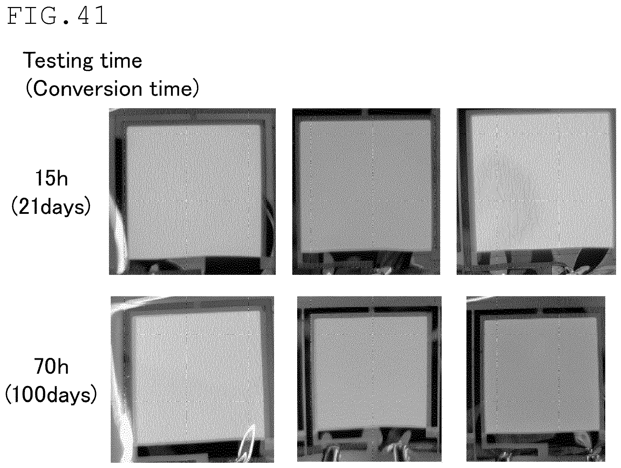

(Step 6) The organic electroluminescence device produced was stored in an environment with a temperature of 85°C and a humidity of 85%, and the stability in the atmosphere of the device was evaluated. The results are shown in FIG. 41. No dark spots were observed even after 100 days of storage, and no erosion from the edge of the emitting surface was observed as a non-emitting portion.

### (Comparative Example 5)

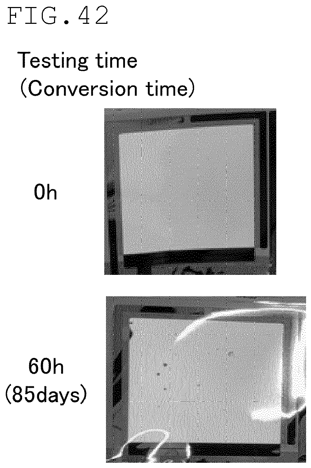

An organic electroluminescence device was produced as in Example 12, except that (Step 2) was replaced by (Step 2-C2) below and (Step 3) was replaced by (Step 3-C2) below. As (Step 6), the organic electroluminescence device produced was stored in an environment with a temperature of 85°C and a humidity of 85%, and the stability in the atmosphere of the device was evaluated. The results are shown in FIG. 42. Dark spots were observed after 100 days of storage, and erosion from the edge of the emitting surface was also observed as a non-emitting portion.

(Step 2-C2) Next, the substrate subjected to the treatment in (Step 1) was placed in a vacuum apparatus, the pressure was reduced to 5 × 10⁻⁵ Pa or lower, and a 10-nm thick silver layer was formed thereon as a cathode by vacuum deposition. Furthermore, sputtering was performed under introduced argon and oxygen to form a zinc oxide layer (a metal oxide layer) having a thickness of about 2 nm. Thereafter, a 10-nm thick silver thin film was formed by deposition.

(Step 3-C2) Next, polyethyleneimine (available from Nippon Shokubai Co., Ltd., EPOMIN P1000, molecular weight 70,000) was diluted with ethanol to 0.05% by weight, and the dilution was spin-coated to form a film having a thickness of 1 to 2 nm as an organic buffer layer 13. Thereafter, the workpiece was placed in a vacuum apparatus again, and the pressure therein was reduced to 5 × 10⁻⁵ Pa or lower. Then, the following layers were stacked: a 15-nm thick electron transport layer 7 made of an electron transport material KHLHEI-02 available from Chemipro Kasei Kaisha, Ltd.; a 15-nm thick hole blocking layer 12 made of KHLHS-04 available from Chemipro Kasei Kaisha, Ltd.; a 35-nm thick emitting layer 8 formed by co-evaporation of KHLHS-03, KHLHS-04, and KHLDG-01 each available from Chemipro Kasei Kaisha, Ltd.; and a 25-nm thick hole transport layer 9 made of KHLHS-03 available from Chemipro Kasei Kaisha, Ltd.

Example 12 shows that the stability in the atmosphere confirmed in the devices including the glass is also confirmed in the film device. In addition, as compared with Comparative Example 5, almost no growth of dark spots was observed in Example 12. This demonstrates that resistance to the atmosphere is high in Example 12.

### <Organic electroluminescence device according to second aspect of the present invention>

### (Example 13)

(Step 1) A resist material was applied to a barrier film (PT7/25GT3: thickness 25 um, WVTR: 4 × 10⁻³ g/m²/day) available from OIKE & Co., Ltd. to form a film, and a 24-nm thick zinc oxide film was formed with a sputtering device as a metal oxide layer 4.

(Step 2) Next, the substrate subjected to the treatment in (Step 1) was placed in a vacuum apparatus, the pressure was reduced to 5 × 10⁻⁵ Pa or lower, and a layer having a total thickness of 10 nm containing silver and magnesium in a volume ratio of 70:30 was formed thereon as a cathode 3 by vacuum deposition.

(Step 3) Next, the following layers were stacked by vacuum deposition: a 3-nm thick organic electron injection layer 6 made of an organic electron injection layer material represented by the formula (9); a 15-nm thick electron transport layer 7 made of an electron transport material KHLHEI-02 available from Chemipro Kasei Kaisha, Ltd.; a 15-nm thick hole blocking layer 12 made of KHLHS-04 available from Chemipro Kasei Kaisha, Ltd.; a 35-nm thick emitting layer 8 formed by co-evaporation of KHLHS-03, KHLHS-04, and KHLDG-01 each available from Chemipro Kasei Kaisha, Ltd.; and a 25-nm thick hole transport layer 9 made of KHLHS-03 available from Chemipro Kasei Kaisha, Ltd.

(Step 4) Next, a hole injection layer 10 was formed on the hole transport layer 9. Specifically, a 10-nm thick molybdenum oxide film was formed by vacuum deposition, which is vapor deposition.

(Step 5) Finally, an anode 11 was formed on the hole injection layer 10. Specifically, a 200-nm thick aluminum film was formed by vacuum deposition. Furthermore, the workpiece was sealed with a barrier film (thickness: 25 um, WVTR: 4 × 10⁻³ g/m²/day) available from OIKE & Co., Ltd.

Through (Step 1) to (Step 5), a film organic electroluminescence device having a structure shown in FIG. 10 was produced. The thickness was about 98 um.

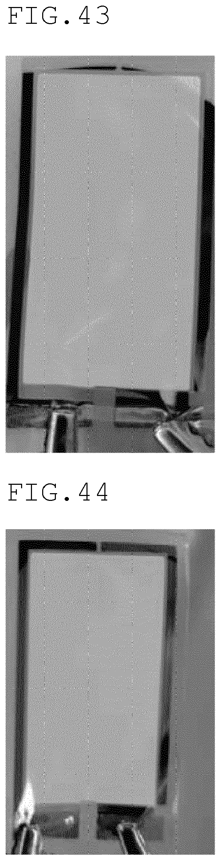

(Step 6) The organic electroluminescence device produced was stored in an environment with a temperature of 85°C and a humidity of 85%, and the stability in the atmosphere of the device was evaluated. The results are shown in FIG. 43. No dark spots were observed even after 65 hours.

### (Example 14)

An organic electroluminescence device was produced as in Example 13, except that (Step 2) was changed to (Step 2-2) below. As (Step 6), the stability in the atmosphere of the organic electroluminescence device was evaluated as in Example 13. The result was shown in FIG. 44. No dark spots were observed even after 65 hours.

(Step 2-2) Next, the substrate subjected to the treatment in (Step 1) was placed in a vacuum apparatus, the pressure was reduced to 5 × 10⁻⁵ Pa or lower, and a layer having a total thickness of 10 nm containing silver and magnesium in a volume ratio of 95:5 was formed thereon as a cathode 3 by vacuum deposition.

### (Example 15)

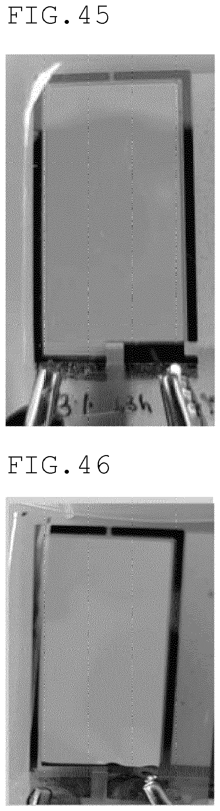

An organic electroluminescence device was produced as in Example 13, except that (Step 2) was changed to (Step 2-3) below. As (Step 6), the stability in the atmosphere of the organic electroluminescence device was evaluated as in Example 13. The result was shown in FIG. 45. No dark spots were observed even after 65 hours.

(Step 2-3) Next, the substrate subjected to the treatment in (Step 1) was placed in a vacuum apparatus, the pressure was reduced to 5 × 10⁻⁵ Pa or lower, and a layer having a total thickness of 10 nm containing silver and magnesium in a volume ratio of 97:3 was formed thereon as a cathode 3 by vacuum deposition.

### (Example 16)

An organic electroluminescence device was produced as in Example 13, except that (Step 2) was changed to (Step 2-4) below. As (Step 6), the stability in the atmosphere of the organic electroluminescence device was evaluated as in Example 13. The result was shown in FIG. 46. No dark spots were observed even after 65 hours.

(Step 2-4) Next, the substrate subjected to the treatment in (Step 1) was placed in a vacuum apparatus, the pressure was reduced to 5 × 10⁻⁵ Pa or lower, and a layer having a total thickness of 25 nm containing silver and magnesium in a volume ratio of 95:5 was formed thereon as a cathode 3 by vacuum deposition.

### (Example 17)

An organic electroluminescence device was produced as in Example 13, except that (Step 2) was changed to (Step 2-5) below. As (Step 6), the stability in the atmosphere of the organic electroluminescence device was evaluated as in Example 13. No dark spots were observed even after 65 hours.

(Step 2-5) Next, the substrate subjected to the treatment in (Step 1) was placed in a vacuum apparatus, the pressure was reduced to 5 × 10⁻⁵ Pa or lower, and a layer having a total thickness of 35 nm containing silver and magnesium in a volume ratio of 95:5 was formed thereon as a cathode 3 by vacuum deposition.

### (Comparative Example 6)

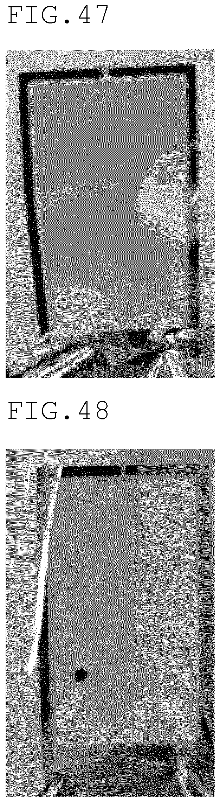

An organic electroluminescence device was produced as in Example 13, except that (Step 2) was changed to (Step 2-6) below. As (Step 6), the stability in the atmosphere of the organic electroluminescence device was evaluated as in Example 13. The result was shown in FIG. 47. Dark spots were observed after 65 hours.

(Step 2-6) Next, the substrate subjected to the treatment in (Step 1) was placed in a vacuum apparatus, the pressure was reduced to 5 × 10⁻⁵ Pa or lower, and a layer having a total thickness of 10 nm containing silver and magnesium in a volume ratio of 50:50 was formed thereon as a cathode 3 by vacuum deposition.

### (Comparative Example 7)

An organic electroluminescence device was produced as in Example 13, except that (Step 2) was changed to (Step 2-7) below. As (Step 6), the stability in the atmosphere of the organic electroluminescence device was evaluated as in Example 13. After 65 hours, no luminescence was confirmed.

(Step 2-7) Next, the substrate subjected to the treatment in (Step 1) was placed in a vacuum apparatus, the pressure was reduced to 5 × 10⁻⁵ Pa or lower, and a layer having a total thickness of 10 nm containing silver and magnesium in a volume ratio of 99:1 was formed thereon as a cathode 3 by vacuum deposition.

### (Comparative Example 8)

An organic electroluminescence device having a structure shown in FIG. 11 was produced as in Example 13, except that (Step 2) was changed to (Step 2-8) below. As (Step 6), the stability in the atmosphere of the organic electroluminescence device was evaluated as in Example 13. The result was shown in FIG. 48. Many dark spots were observed after 65 hours.

(Step 2-8) Next, the substrate subjected to the treatment in (Step 1) was placed in a vacuum apparatus, the pressure was reduced to 5 × 10⁻⁵ Pa or lower, and a 9-nm thick silver layer and a 1-nm thick magnesium layer were formed thereon respectively as a cathode 3 and a metal electron injection layer 5 by vacuum deposition. Thereby, the device wherein magnesium was separated from the cathode as the metal electron injection layer 5, and stacked was obtained.

### (Comparative Example 9)

An organic electroluminescence device was produced as in Example 13, except that (Step 2) was changed to (Step 2-9) below. As (Step 6), the stability in the atmosphere of the organic electroluminescence device was evaluated as in Example 13. No luminescence was confirmed after 65 hours.

(Step 2-9) Next, the substrate subjected to the treatment in (Step 1) was placed in a vacuum apparatus, the pressure was reduced to 5 × 10⁻⁵ Pa or lower, and a layer having a total thickness of 45 nm containing silver and magnesium in a volume ratio of 95:5 was formed thereon as a cathode 3 by vacuum deposition.

The initial luminescence characteristics of the devices of Examples 13 to 17 and Comparative Examples 6 to 9 are shown in the following Table 1.

**[Table 1]**

| | Example 13 | Example 14 | Example 15 | Example 16 | Example 17 | Comparative Example 6 | Comparative Example 7 | Comparative Example 8 | Comparative Example 9 |
|---|---|---|---|---|---|---|---|---|---|
| Luminance of device during production under 4 V | Good | Good | Good | Good | Good | Poor | Good | Good | Poor |
| | | | | | | Luminance under 4nV is acceptable, while luminance under 5 V is slightly reduced | | | Luminance is slightly low because of decreased transmissivity |

The results of the test of stability in the atmosphere under the conditions of a temperature of 85°C and a humidity of 85% demonstrate that as for the percentage of magnesium, 30% or lower causes no problem, but 50% or higher is not good in terms of permeability and stability in the atmosphere. As for the lower limit of the percentage of magnesium, even with 1%, the device exhibits characteristics without any problems at the initial stage, while a higher percentage of magnesium is required for the device to have higher stability in the atmosphere. As shown in Example 15, with 3% of magnesium, the device has high stability in the atmosphere. No problem occurs in both the initial characteristics and stability in the atmosphere up to a film thickness of 35 nm, while with a film thickness of 45 nm, dark spots were observed in the evaluation of stability in the atmosphere. As shown in the results of the examples, the lower limit of the film thickness of not more than 10 nm was acceptable. As for the film thickness, a film with a thickness of 7 nm or less is confirmed to be formed without any variation in thickness.

### (Example 18)

An organic electroluminescence device was produced as in Example 12, except that (Step 1) was changed to (Step 1-D) below and (Step 2) was changed to (Step 2-D) below. As in Example 12, the thickness of the device was about 98 um. (Step 6-D) below was performed as (Step 6) for evaluation.

(Step 1-D) A resist material was applied to a barrier film (PT7/25GT3: thickness 25 um, WVTR: 4 × 10⁻³ g/m²/day) available from OIKE & Co., Ltd. to form a film. In this example, the metal oxide layer 4 was omitted.

(Step 2-D) Next, a 20-nm thick layer was formed as a layer serving as a cathode 3 and a metal electron injection layer 5 by sputtering using a sputtering target made of silver and zinc in a volume ratio of 9:1.

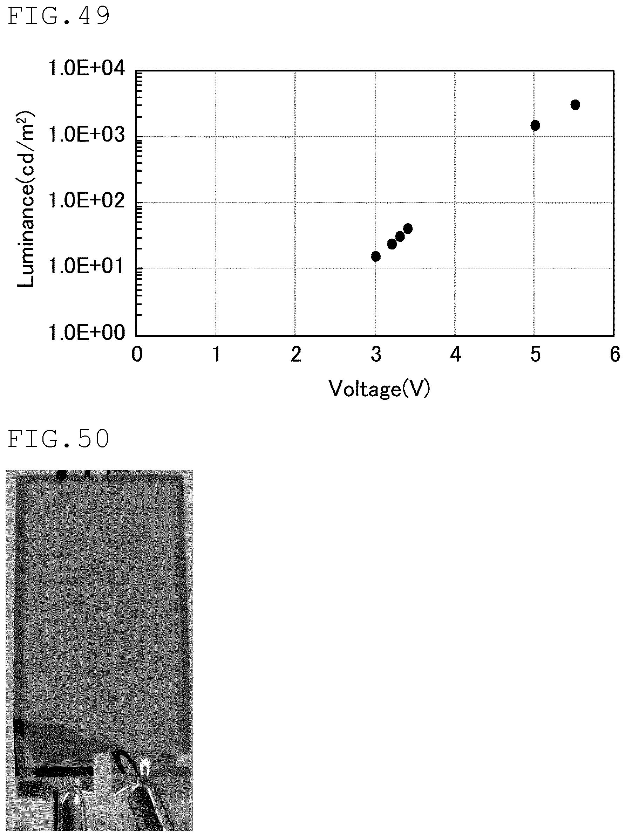

(Step 6-D) "Model 2400 SourceMeter" available from Keithley Instruments was used to apply a voltage to the device and to measure the current. Luminance was measured using "BM-3AR" available from Topcon Corporation. The measurement was performed in air. The results are shown in FIG. 49.

The organic electroluminescence device produced was stored in an environment with a temperature of 85°C and a humidity of 85%, and the stability in the atmosphere of the device was evaluated. The results after 87 hours are shown in FIG. 50. No dark spots were observed even after 120 hours of storage, and no erosion from the edge of the emitting surface was observed as a non-emitting portion.

### REFERENCE SIGNS LIST

1: organic EL device, 2: substrate, 3: cathode, 4: metal oxide layer, 5: metal electron injection layer, 6: organic electron injection layer, 7: electron transport layer, 8: emitting layer, 9: hole transport layer, 10: hole injection layer, 11: anode, 12: hole blocking layer, 13: organic buffer layer

## Claims

1. An organic electroluminescence device at least comprising:
a pair of electrodes; and
an organic layer between the electrodes,
the organic electroluminescence device comprising
a metal electron injection layer on the cathode and an organic electron injection layer on the metal electron injection layer,
the metal electron injection layer containing a metal species and the organic electron injection layer containing an organic material, the metal species and the organic material being capable of coordination.

2. The organic electroluminescence device according to claim 1,
wherein the metal species, or when the metal species includes multiple metal species, all the metal species in the metal electron injection layer has a work function of 4.0 eV or higher.

3. The organic electroluminescence device according to claim 1 or 2,
wherein the metal electron injection layer contains at least one selected from the group consisting of zinc, copper, and aluminum.

4. The organic electroluminescence device according to any one of claims 1 to 3,
wherein the metal electron injection layer has a thickness of 5 nm or less.

5. The organic electroluminescence device according to any one of claims 1 to 4,
wherein the organic material in the organic electron injection layer is a compound having a nitrogen-containing substituent.

6. The organic electroluminescence device according to any one of claims 1 to 5,
wherein the organic material in the organic electron injection layer is a compound having a fused ring structure containing nitrogen-containing heterocyclic ring.

7. The organic electroluminescence device according to any one of claims 1 to 6,
wherein the organic material in the organic electron injection layer is a compound having a structure represented by the following formula (1): 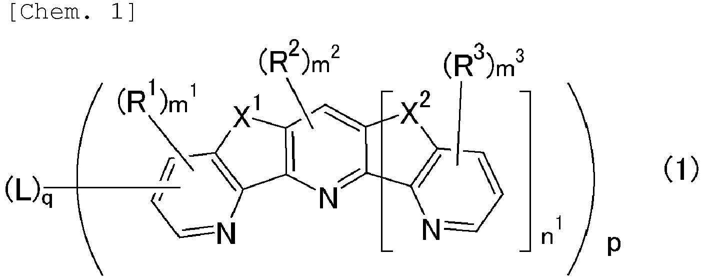 wherein X¹ and X² are the same as or different from each other and are each a nitrogen atom optionally having a substituent, an oxygen atom optionally having a substituent, a sulfur atom optionally having a substituent, or a divalent linking group optionally having a substituent; L is a direct bond or a linking group having a valence of p; n¹ is the number of 0 or 1; p is the number of 1 to 4; q is the number of 0 or 1, with q being 0 when p is 1; R¹ to R³ are the same as or different from each other and are each a monovalent substituent; m¹ to m³ are the same as or different from each other and are each the number of 0 to 3; R¹ to R³ are each optionally combined with X¹ or X² to form a ring structure; and when R¹ includes multiple R¹'s, R¹'s are optionally combined to each other to form a ring structure, with the same applying to R² and R³.

8. The organic electroluminescence device according to any one of claims 1 to 6,
wherein the organic material in the organic electron injection layer is a compound having a structure, as a skeletal structure, represented by any of the following formulas (5-66) to (5-68):

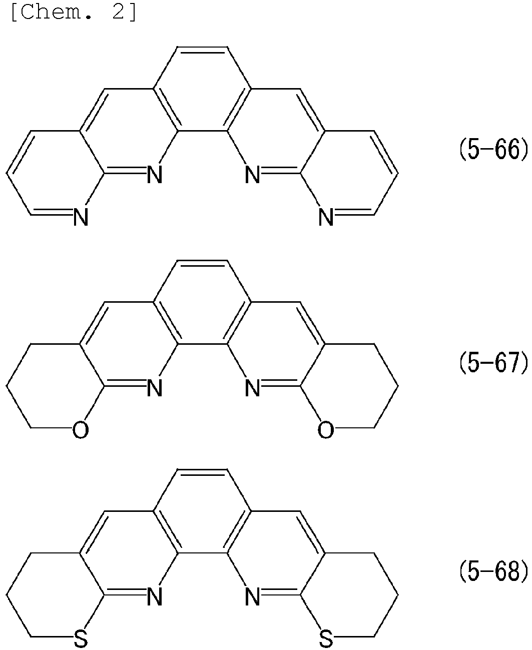

9. The organic electroluminescence device according to any one of claims 1 to 6,
wherein the organic material in the organic electron injection layer is a hexahydropyrimidopyrimidine compound having a structure represented by the following formula (6) : 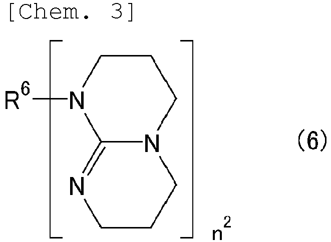 wherein R⁶ is an aromatic hydrocarbon group optionally having a substituent, an aromatic heterocyclic group optionally having a substituent, an arylalkylene group optionally having a substituent, a divalent to tetravalent acyclic or cyclic hydrocarbon group optionally having a substituent, a group of a combination of two or more of these groups, or a group of a combination of a nitrogen atom and one or more of these groups; and n² is an integer of 1 to 4.

10. The organic electroluminescence device according to any one of claims 1 to 9,
wherein the cathode has a work function of 4.0 eV or higher.

11. The organic electroluminescence device according to any one of claims 1 to 10,
wherein the cathode is on a substrate.

12. The organic electroluminescence device according to any one of claims 1 to 3 and 5 to 11,
wherein the metal electron injection layer is obtained by mixing with a cathode constituent material.

13. An organic electroluminescence device at least comprising:
a pair of electrodes; and
an organic layer between the electrodes,
the cathode being a layer having an average thickness of 40 nm or less and containing magnesium and at least one of silver or aluminum, with a percentage by volume of magnesium being 2% or higher and 30% or lower,
the organic electroluminescence device comprising
an organic electron injection layer being adjacent to the cathode and containing an organic material capable of coordinating to a metal atom.

14. The organic electroluminescence device according to claim 13,
wherein the organic material in the organic electron injection layer is a compound having a nitrogen-containing substituent.

15. The organic electroluminescence device according to claim 13 or 14,
wherein the organic material in the organic electron injection layer is a compound having a fused ring structure containing nitrogen-containing heterocyclic ring.

16. The organic electroluminescence device according to any one of claims 13 to 15,
wherein the organic material in the organic electron injection layer is a compound having a structure represented by the following formula (1): 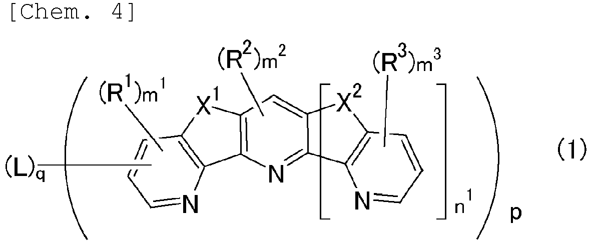 wherein X¹ and X² are the same as or different from each other and are each a nitrogen atom optionally having a substituent, an oxygen atom optionally having a substituent, a sulfur atom optionally having a substituent, or a divalent linking group optionally having a substituent; L is a direct bond or a linking group having a valence of p; n¹ is the number of 0 or 1; p is the number of 1 to 4; q is the number of 0 or 1, with q being 0 when p is 1; R¹ to R³ are the same as or different from each other and are each a monovalent substituent; m¹ to m³ are the same as or different from each other and are each the number of 0 to 3; R¹ to R³ are each optionally combined with X¹ or X² to form a ring structure; and when R¹ includes multiple R¹'s, R¹'s are optionally combined to each other to form a ring structure, with the same applying to R² and R³.

17. The organic electroluminescence device according to any one of claims 13 to 15,
wherein the organic material in the organic electron injection layer is a compound having a structure, as a skeletal structure, represented by any of the following formulas (5-66) to (5-68):

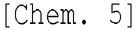

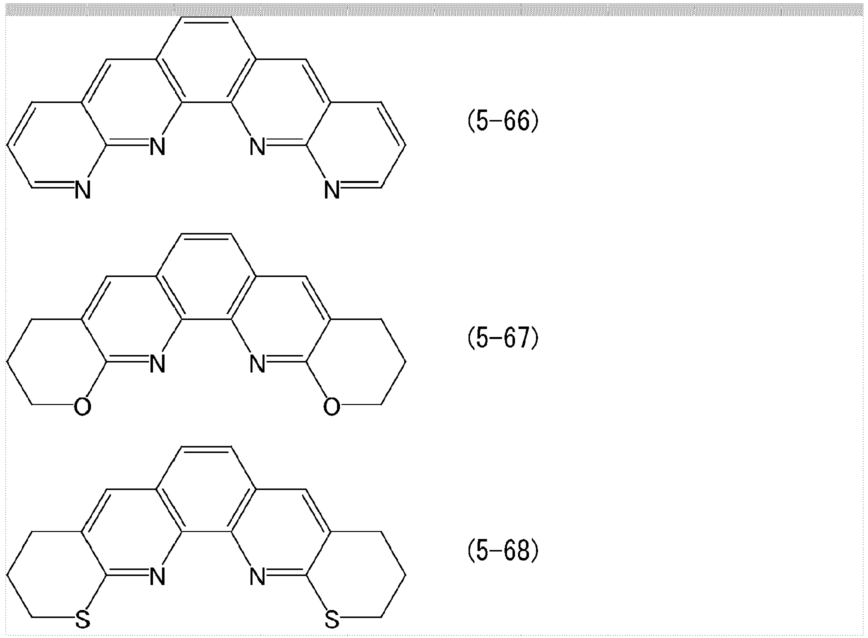

18. The organic electroluminescence device according to any one of claims 13 to 15,
wherein the organic material in the organic electron injection layer is a hexahydropyrimidopyrimidine compound having a structure represented by the following formula (6) : 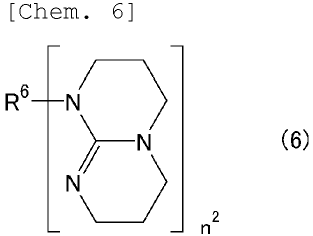 wherein R⁶ is an aromatic hydrocarbon group optionally having a substituent, an aromatic heterocyclic group optionally having a substituent, an arylalkylene group optionally having a substituent, a divalent to tetravalent acyclic or cyclic hydrocarbon group optionally having a substituent, a group of a combination of two or more of these groups, or a group of a combination of a nitrogen atom and one or more of these groups; and n² is an integer of 1 to 4.

19. The organic electroluminescence device according to any one of claims 13 to 18,
wherein the cathode is on a substrate.

20. A display comprising the organic electroluminescence device according to any one of claims 1 to 19.

21. A lighting system comprising the organic electroluminescence device according to any one of claims 1 to 19.

22. A method for producing an organic electroluminescence device at least comprising a pair of electrodes and an organic layer between the electrodes, the method comprising:
forming a metal electron injection layer adjacent to the cathode and containing a metal species; and
forming on the metal electron injection layer an organic electron injection layer containing an organic material capable of coordinating to the metal species in the metal electron injection layer.

23. A method for producing an organic electroluminescence device comprising at least a pair of electrodes and an organic layer between the electrodes, the method comprising:
forming a cathode having an average thickness of 40 nm or less and containing magnesium and at least one of silver or aluminum, a percentage by volume of magnesium being 2% or higher and 30% or lower; and
forming on the cathode an organic electron injection layer containing an organic material capable of coordinating to a metal atom in the cathode.
